# EUROPEAN PATENT APPLICATION

(11) **EP 3 569 710 A1**
(43) Date of publication of application: **20.11.2019**
(21) Application number: 18172731.4
(22) Date of filing: 16.05.2018
(51) Int. Cl.: C12N 15/10, C12Q 1/689, C12N 15/74

(54) **BACTERIAL VECTORS FOR GENETIC MANIPULATION OF BACTERIA**

(71) Applicant: BioVersys AG, 4057 Basel (CH)
(72) Inventor: Kemmer, Christian, 4125 Riehen (CH); Trebosc, Vincent, 68330 Huningue (FR); Pieren, Michel, 4106 Therwil (CH); Gitzinger, Marc, 5080 Laufenburg (CH); Schellhorn, Birgit, 79576 Weil am Rhein (DE)
(74) Representative: Latscha Schöllhorn Partner AG

(57) **Abstract**

The present invention relates to the field of bacterial vectors and methods for genetic manipulation of bacteria. In particular, the present invention relates to a vector for genetic manipulation of a bacterium, wherein said vector comprises (a) a non-antibiotic selection marker gene cassette, (b) an origin of replication, wherein said origin of replication is not capable of inducing replication of said vector in said bacterium, and (c) a restriction endonuclease gene, a recognition site of a restriction endonuclease encoded by said restriction endonuclease gene, and a second regulatory sequence. Further, the invention relates to a bacterial host cell comprising said vector, a method for genetic manipulation of bacteria using the vector of the invention, and methods for selecting bacterial host cells.

## Description

The present invention relates to the field of bacterial vectors and methods for genetic manipulation of bacteria. In particular, the present invention relates to a vector for genetic manipulation of a bacterium, wherein said vector comprises (a) a non-antibiotic selection marker gene cassette, (b) an origin of replication, wherein said origin of replication is not capable of inducing replication of said vector in said bacterium, and (c) a restriction endonuclease gene, a recognition site of a restriction endonuclease encoded by said restriction endonuclease gene, and a second regulatory sequence. Further, the invention relates to a bacterial host cell comprising said vector, a method for genetic manipulation of bacteria using the vector of the invention, and methods for selecting bacterial host cells.

### RELATED ART

The emergence and prevalence of multidrug resistant (MDR) pathogenic bacteria poses a serious threat to human and animal health globally. Nosocomial infections and common ailments such as pneumonia, wound, urinary tract, and bloodstream infections are becoming more challenging to treat due to the rapid spread of MDR pathogenic bacteria. According to recent reports by the World Health Organization (WHO) and Centers for Disease Control and Prevention (CDC), there is an unprecedented increase in the occurrence of MDR infections worldwide. The rise in these infections has generated an economic strain worldwide, prompting the WHO to endorse a global action plan to improve awareness and understanding of antimicrobial resistance. This health crisis necessitates an immediate action to target the underlying mechanisms of drug resistance in bacteria (Krishnamurthy et al., Bacterial genome engineering and synthetic biology: combating pathogens, BMC Microbiol. 2016; 16: 258).

A number of methods have been developed for engineering bacterial genomes with varying degrees of efficiency, specificity and broad host applicability. Most often, the bacterial genome editing is carried out to knock-out genes, knock-in genes or introduce mutations in the bacterial genome. Though most of these methods were developed in *E. coli,* in the last decade there has been a rapid development and expansion of these tools to a broad range of bacterial hosts (Krishnamurthy *et al.,* 2016, *op. cit.*)*.*

Bacterial chromosomal modifications have greatly aided in better comprehension of bacterial pathogenesis and virulence mechanisms. Among the genome engineering methods, the utilization of the λ-Red recombinase system for insertions, deletions or point mutations of the genome has been very popular. Pioneered by Murphy et al. (Use of bacteriophage lambda recombination functions to promote gene replacement in Escherichia coli, J Bacteriol. 1998;180(8):2063-2071) and later modified by Datsenko et al. (One-step inactivation of chromosomal genes in Escherichia coli K-12 using PCR products, Proc Natl Acad Sci USA. 2000;97(12):6640-6645), this method involves the introduction of single- or double-stranded DNA with chromosomal homology regions for recombination (Sawitzke et al., Probing cellular processes with oligo-mediated recombination and using the knowledge gained to optimize recombineering, J Mol Biol. 2011;407(1):45-59).

Datsenko *et al.,* 2000 (*op. cit*.) developed a procedure based on the Red system by which chromosomal genes in *Escherichia coli* can be disrupted and PCR primers provide the homology to targeted gene(s). In this procedure, recombination requires the phage 1 Red recombinase, which is synthesized under the control of an inducible promoter on a curable, low copy number plasmid. To demonstrate the utility of this approach, PCR products were generated by using primers with 36- to 50-nt extensions that are homologous to regions adjacent to the gene to be inactivated and template plasmids carrying antibiotic resistance genes that are flanked by FRT (FLP recognition target) sites. This is accomplished by Red-mediated recombination in these flanking homologies. After selection, the resistance gene can also be optionally eliminated by using a helper plasmid expressing the FLP recombinase, which acts on the directly repeated FRT (FLP recognition target) sites flanking the resistance gene. The Red and FLP helper plasmids can be cured by growth at 37°C because they are temperature-sensitive replicons.

In a further approach, Amin *et al.,* 2013 developed a method for generating marker-less gene deletions in multidrug-resistant *Acinetobacter. Acinetobacter baumannii (A. baumannii)* is an important nosocomial pathogen that has become increasingly resistant to multiple antibiotics. Genetic manipulation of MDR *A. baumannii* is useful especially for defining the contribution of each active efflux mechanism in multidrug resistance. A tellurite-resistant (sacB+, xylE+) suicide vector, pMo130-*TelR, was created for deleting the adeFGH and adeIJK operons in two clinical MDR A.* baumannii. Using a two-step selection, plasmid insertion recombinants (first-crossover) were selected for tellurite resistance and the deletion mutants (second-crossover) were then selected for loss of sacB (Amin et al., A Method for generating marker-less gene deletions in multidrug-resistant A. baumannii, BMC Microbiology 2013, 13:158).

Thus, there is a high need for a platform that allows genetic manipulations of drug-resistant clinical pathogen strains, independent of their drug resistance profile.

### SUMMARY OF THE INVENTION

The invention presents herein a new approach for modifying nucleic acid or nucleotide sequences in bacteria.

In a first aspect, the invention relates to a vector for manipulation of a genome of a bacterium, wherein said vector comprises
(a) at least one non-antibiotic selection marker gene cassette comprising at least one non-antibiotic selection marker gene and a first regulatory sequence, wherein said first regulatory sequence is operatively linked to said at least one non-antibiotic selection marker gene,
(b) an origin of replication, wherein said origin of replication is not capable of inducing replication of said vector in said bacterium, and
(c) a restriction endonuclease gene, a recognition site of a restriction endonuclease encoded by said restriction endonuclease gene, and a second regulatory sequence, wherein said second regulatory sequence is operatively linked to said restriction endonuclease gene.

In a second aspect, the invention relates to a bacterium comprising the vector of the invention.

In a further aspect, the invention relates to a method for genetic manipulation of bacteria comprising the steps of:
(a) transferring the vector of the invention, comprising said first and said second nucleotide sequences and optionally said third nucleotide sequence, into bacterial host cells,
(b) culturing the bacterial host cells in a first medium,
(c) selecting from the cultured cells of step (b), bacterial host cells having the vector integrated into their genome,
(d) culturing the selected bacterial host cells of step (c) in a second medium, and
(e) counter-selecting from the cultured cells of step (d), bacterial host cells in which a target DNA sequence is eliminated from the bacterial genome, or in which said third nucleotide sequence is integrated into the bacterial genome.

In a further aspect, the invention relates to a method for selecting bacterial host cells comprising the steps of:
(a) transferring a vector into bacterial host cells, wherein said vector comprises
   - a thiopurine S-methyltransferase (tpm) gene, and
   - a regulatory sequence operatively linked to said tpm gene,
(b) culturing said bacterial host cells in a medium containing more than 100 µg/ml tellurite,
(c) selecting bacterial host cells that survived the tellurite concentration of step (b).

In a further aspect, the invention relates to a method for selecting bacterial host cells comprising the steps of:
(a) transferring a vector into bacterial host cells, wherein said vector comprises a marker gene,
(b) culturing the bacterial host cells in medium containing a compound selected from the group consisting of a bicyclic compound substituted with at least one hydroxyl group or at least one amino group; a branched fatty acid having a chain length of more than C13; and an unsaturated fatty acid having a chain length of more than C14, and
(c) isolating bacterial host cells that express said marker gene or that do not express said marker gene.

Genetic manipulation of multi drug-resistant clinical pathogen strains with conventional methods is very difficult, because these methods use antibiotic resistance cassettes to select for the genetic modifications. However, multi-, extensively-, or even pandrug resistant clinical isolates have acquired a multitude of resistance mechanisms to overcome the poisonous action of antibiotics. Since some pathogens are already resistant to many, if not all antibiotics, the mutant selection via additional introduction of antibiotic resistance cassettes is not possible.

In order to investigate the function of putative drug targets in clinically problematic drug-resistant isolates of bacteria, the inventors developed a technology platform enabling the genome modification of bacteria, especially drug-resistant strains. The platform is based on vectors and methods combining a non-antibiotic selection marker gene cassette, an origin of replication which is not capable of inducing replication of said vector in the bacterium to be genetically engineered, and a cassette comprising a restriction endonuclease gene and a recognition site of a restriction endonuclease encoded by said restriction endonuclease gene.

The vectors and methods of the invention allow the genetic engineering of clinical pathogens and laboratory strains, independent of their drug resistance profile. In addition, the described method is much faster (3 days) than the usually taken λred-system, e.g., as developed by Datsenko *et al.* 2000 (*op. cit.*), which takes around 10 days, uses multiple antibiotic resistance cassettes as selection markers, requires 2 rounds of plasmid curing, and leaves a genomic scar (i.e. additional nucleotides) in the genome at the mutation site that may introduce unwanted side effects. The inventors developed a vectors and method for the introduction of genome modifications free of genomic scars in drug resistant clinical isolates.

### DESCRIPTION OF THE FIGURES

**FIGURE 1****:** Cloning vector pCK452
**FIGURE 2****:** Knock-out vector pCK476, used for ompR in *Klebsiella pneumoniae*
**FIGURE 3****:** Nucleotide sequence designs for knock-out (KO), knock-in (KI) and site directed mutagenesis (SDM). Target gene (T), upstream (up) and downstream (do) flanking sequences (FS), fragment to be inserted by knock-in (I), nucleotide variation generated by site directed mutagenesis (V).
**FIGURE 4****:** Genomic knock-out of a target gene (1^{st} recombination); upstream integration (Fig. 4A, left); downstream integration (Fig. 4A, right); followed by PCR screen for genomic plasmid integration using primers P1+P2. P1 anneals in genome upstream of flanking site; P2 anneals in vector; P3 anneals in genome downstream of flanking site; P4 and P5 anneal in target gene. Genomic DNA (gDNA), target gene (T), upstream (up) and downstream (do) flanking sequences (FS).
   Selection with 200 µg/mL anhydrotetracycline (aTc) to induce SceI counter-selection (Fig. 4B); 50% successful target gene knock-out (KO); 50% wildtype revertant (WT); followed by PCR screen for gene deletion:
   Primer P1+P3: gene knock-out: approx. 1.6kb, wildtype: 1.6kb + gene of interest;
   Primer P4+P5: gene deletion: no PCR product, wildtype: PCR product for target gene (T).

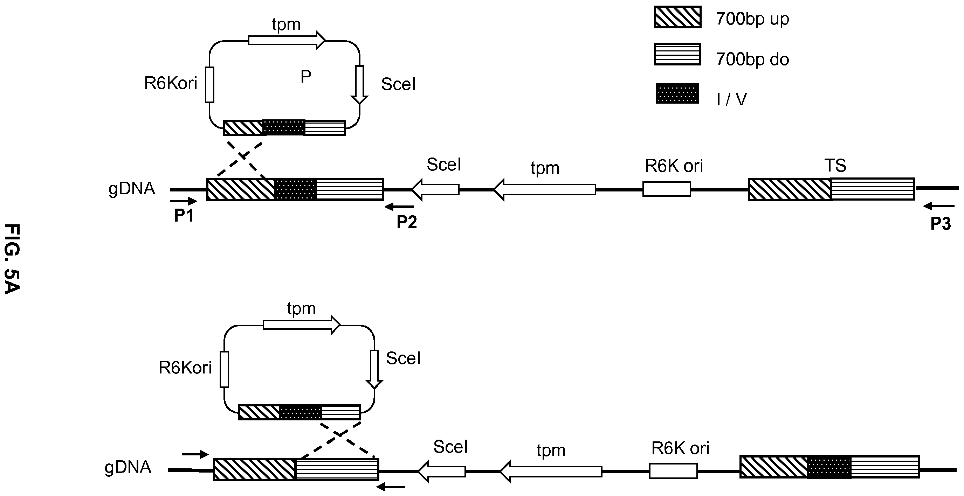

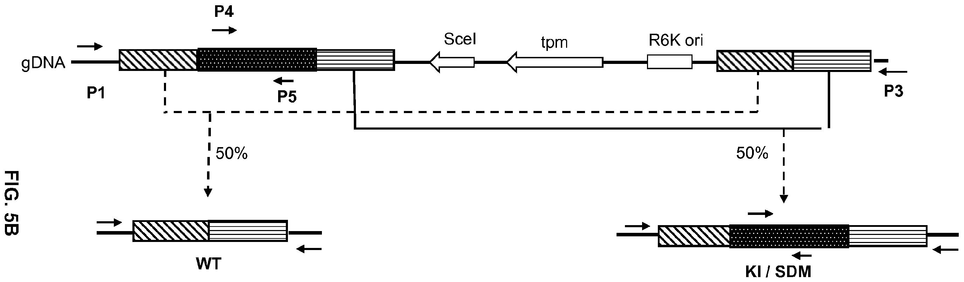

**FIGURE 5****:** Genomic knock-in (KI) and site directed mutagenesis (SDM) at a target site (TS) (1^{st} recombination); upstream integration (Fig. 5A, left); downstream integration (Fig. 5A, right); followed by PCR screen for genomic plasmid integration using primer 1+2. Selection with 200 µg/mL aTc to induce SceI counter-selection (Fig. 5B); 50% successful target gene knock-in (KI) or site-directed mutagenesis (SDM) of nucleotides; 50% wildtype revertant. For site directed mutagenesis, the nucleotide variation (V) was confirmed via Sanger sequencing, but wildtype and mutants cannot be distinguished via different PCR amplicons. For knock-in, successful insertion (I) at the target site was confirmed by PCR with primer P1+P3, and the insert was detected with primer P4+P5.

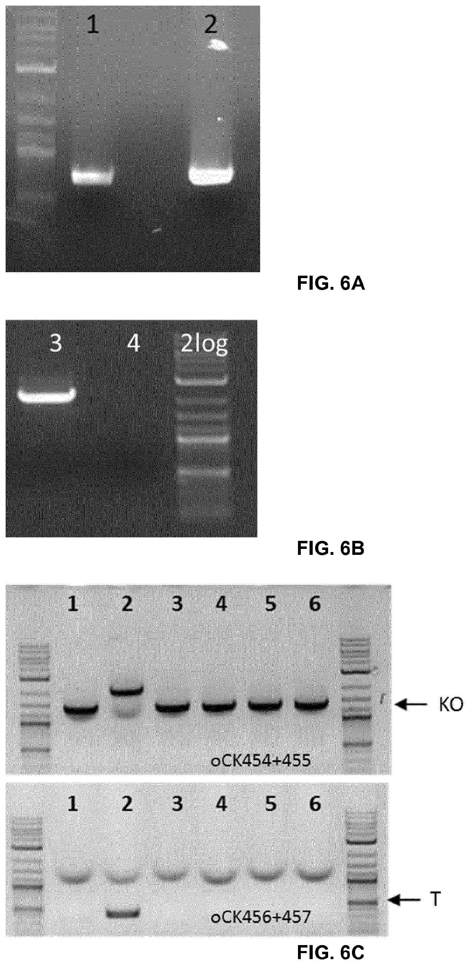

**FIGURE 6****:** PCR-amplification of 700 bp upstream flanking region and 700 bp downstream flanking region; DNA fragments from PCR were separated on an agarose gel; 2-log ladder (NEB); 1: PCR product oCK612+613 (700 bp flank upstream ompR); 2: PCR product oCK614+615 (700 bp flank downstream ompR) (Fig. 6A).
   Colony PCR clone screen to test site directed plasmid integration into genome; DNA fragments from PCR were separated on an agarose gel; 2-log DNA ladder (NEB) 3: PCR product oCK454+354 (successful plasmid integration, 2.3 kb) 4: wildtype cells without integrated plasmid (Fig. 6B).
   Colony PCR screen to confirm successful plasmid removal and ompR deletion, 2-log DNA ladder (NEB). Expected PCR fragment sizes:
   PCR oCK454+455 (flanks + gene): knockout (KO): 1.5kb, wildtype: 2.3kb
   PCR oCK456+457 (ompR target gene (T)): knockout (KO): no product, wildtype: 480bp Clones 1 and 3-6 show successful plasmid removal (1.5 kb) and absence of ompR gene. In contrast, clone 2 is a wildtype revertant and still carries the target gene (Fig. 6C).

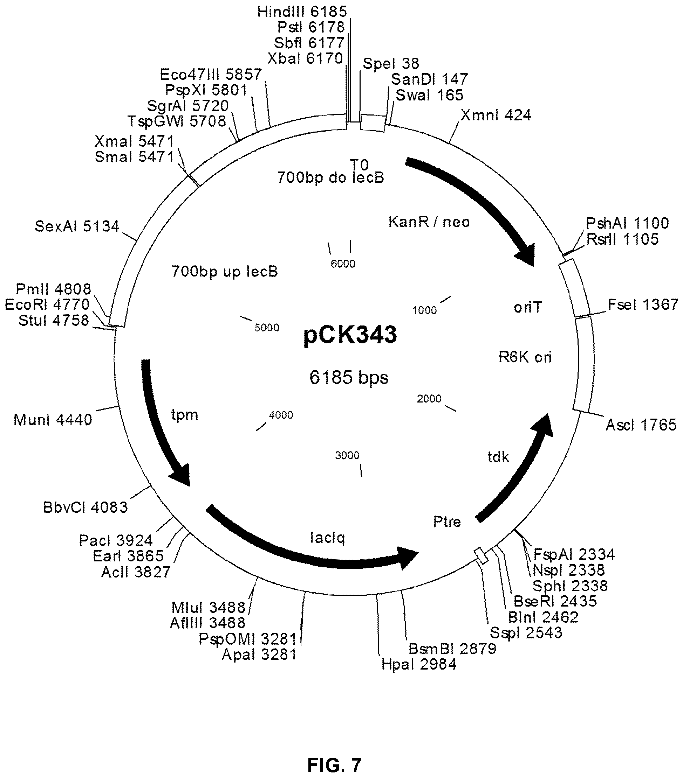

**FIGURE 7****:** pCK343, knock-out vector for lecB in *Pseudomonas aeruginosa*

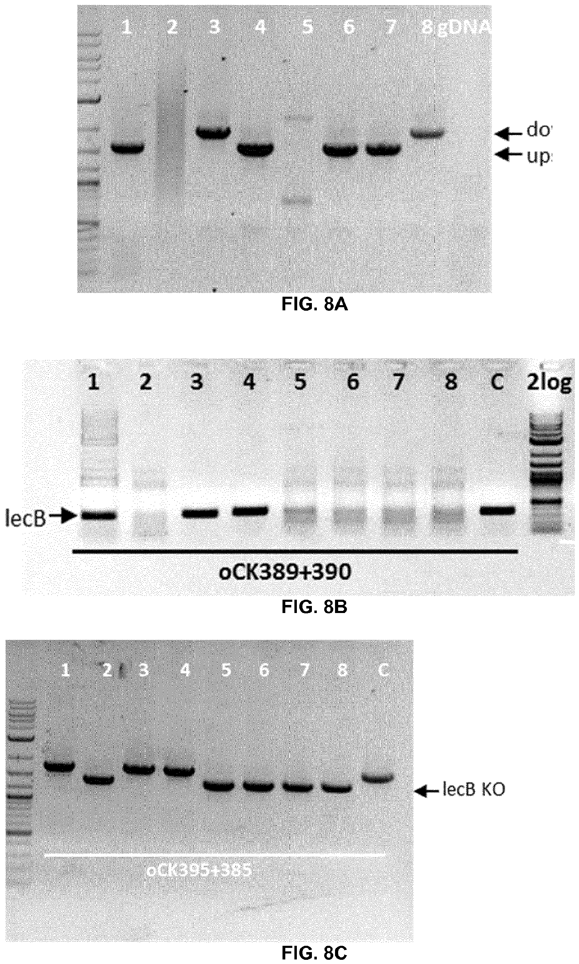

**FIGURE 8****:** Colony PCR screen to test site directed plasmid integration into genome; DNA fragments from PCR were separated on an agarose gel; 2-log DNA ladder (NEB); Clone 1-8: PCR product oCK391+354 (successful plasmid integration upstream (up): 1.6kb, downstream (do): 2.3kb); gDNA: genomic DNA of parental BV93 (shows no band due to absence of plasmid integration) (Fig. 8A).
   Colony PCR screen to confirm successful lecB deletion (KO) and plasmid removal, 2-log DNA ladder (NEB); Expected PCR fragment sizes:
   PCR oCK389+390 (lecB gene): ΔlecB: no product, wildtype: 340bp
   PCR oCK395+395 (flanks + gene): ΔlecB: 1.4kb, wildtype: 1.8kb
   Clones 2, 5-8 show absence of lecB gene and successful plasmid removal (1.4kb) (Fig. 8B). In contrast, clone 1, 3, 4 are wildtype revertants and still carry the lecB gene as the wildtype control (C) (Fig. 8C).

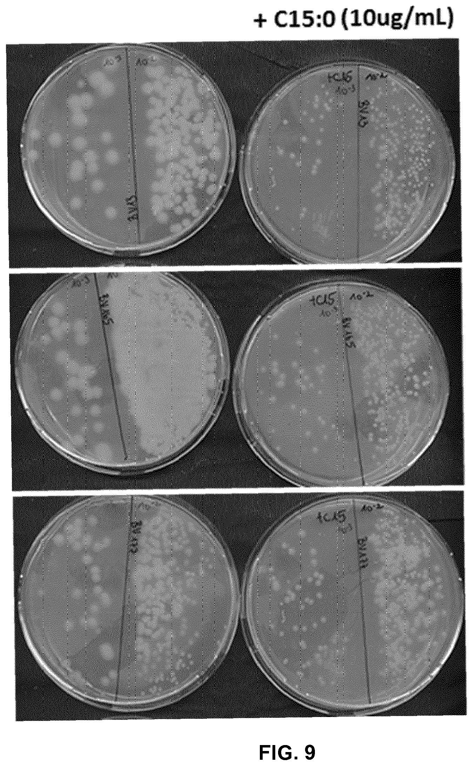

**FIGURE 9****:** Inhibition of the motility of clinical P. aeruginosa strains by 12-Methyl-tetradecanoic acid (C15) on Luria Bertani agar plates.

### DETAILED DESCRIPTION OF THE INVENTION

Throughout this specification and the claims which follow, unless the context requires otherwise, the word "comprise", and variations such as "comprises" and "comprising", are to be understood to imply the inclusion of a stated integer or step or group of integers or steps but not the exclusion of any other integer or step or group of integer or step.

As used in this specification and the appended claims, the singular forms "a", "an", and "the" include plural referents, unless the content clearly dictates otherwise.

The term "about" when used in connection with a numerical value is meant to encompass numerical values within a range having a lower limit that is 10% smaller than the indicated numerical value and having an upper limit that is 10% larger than the indicated numerical value.

In a first aspect, the invention relates to a vector for manipulation of a genome of a bacterium, wherein said vector comprises
(a) at least one non-antibiotic selection marker gene cassette comprising at least one non-antibiotic selection marker gene and a first regulatory sequence, wherein said first regulatory sequence is operatively linked to said at least one non-antibiotic selection marker gene,
(b) an origin of replication, wherein said origin of replication is not capable of inducing replication of said vector in said bacterium, and
(c) a restriction endonuclease gene, a recognition site of a restriction endonuclease encoded by said restriction endonuclease gene, and a second regulatory sequence, wherein said second regulatory sequence is operatively linked to said restriction endonuclease gene.

The terms "genetic manipulation" and "manipulation of a genome" are interchangeably used herein. Further, the terms "genome of a bacterium" and "bacterial genome" are interchangeably used herein and include nucleic acids or nucleotide sequences of a bacteria, derived from a bacteria or included in a bacteria; preferably said nucleic acids are linear and circular DNA (such as linear or circular chromosomes) plasmids and megaplasmids. The terms "genetic engineering" and "genetic manipulation" are interchangeably used herein and refer to modification or mutation of the genome, e.g., by substitution, deletion, insertion, duplication, knock-out, knock-in, site-directed-mutagenesis (SDM) and/or frameshift.

The term "vector" as used herein, refers to a nucleic acid molecule or nucleotide sequence, preferably a DNA molecule or sequence used as a vehicle to artificially introduce genetic material into a host cell. The host cells are herein bacterial cells. In a preferred embodiment the vector of the invention is a plasmid.

The vector of the invention comprises a non-antibiotic selection marker gene cassette comprising at least one non-antibiotic selection marker gene and a first regulatory sequence, wherein said first regulatory sequence is operatively linked to said at least one non-antibiotic selection marker gene. In a preferred embodiment, said non-antibiotic selection marker gene cassette comprises exactly one non-antibiotic selection marker gene and exactly one first regulatory sequence.

As used herein the "regulatory sequence" is a nucleotide sequence comprising at least one regulatory element. Said regulatory element is preferably selected from the group consisting of promoter, enhancer, repressor, silencer, terminator, binding site for regulatory proteins or ribonucleic acids (such as regulatory micro RNA) that promote or inhibit transcription, or a combination thereof. The regulatory sequence does not have to be one continuous nucleotide sequence, but can composed of more than one nucleotide sequence, optionally present as separated or discontinuous nucleotide sequences on the vector. In a preferred embodiment, the regulatory sequence is a bacterial promoter.

In a preferred embodiment, the first regulatory sequence is any kind of constitutive or inducible bacterial promoter. In one preferred embodiment, the first regulatory sequence is a constitutive bacterial promoter. This allows constitutive expression of the at least one non-antibiotic selection marker gene to which the promoter is operatively linked. Constitutive promoters that are suitable for bacterial vectors and bacterial expression are well-known to the skilled person and can for example be found in prokaryotic promoter databases (e.g., http://parts.igem.org/Promoters/Catalog/Constitutive, *Addgene,* or Davis *et al.,* 2011). In a preferred embodiment, said first regulatory sequence is a *Burkholderia cenocepacia* rpsL PCS12 promoter.

In a preferred embodiment, said vector of the invention comprises an origin of transfer (oriT). The oriT is required for the plasmid transfer via conjugation.

The vector of the invention further comprises an origin of replication (oriR), wherein said oriR is not capable of inducing replication of said vector in said bacterium (non-replicative). This means that the type of oriR included in the vector of the invention depends on the type of bacterium that is selected for genetic manipulation. Due to the non-replicative oriR, the extrachromosomal vector of the invention gets lost in the host cells during cell divisions. Only vectors integrated into the genome are reproduced and maintained in the bacterial host cells. In a preferred embodiment, said oriR is non-replicative, since the replicative proteins capable of binding to the oriR are neither encoded on the vector and nor in the bacterium or bacterial species used for genetic manipulation.

In a preferred embodiment, said oriR is non-replicative in *Enterobacteriaceae.* In another preferred embodiment, said oriR is non-replicative in *Enterobacteriaceae* selected from the group consisting of the genera *Escherichia, Klebsiella, Shigella, Enterobacter, Yersinia, Salmonella, Serratia, Citrobacter,* and *Proteus.* In another preferred embodiment, said oriR is non-replicative in bacteria of the genera *Escherichia, Klebsiella, or Shigella.* In another preferred embodiment, said oriR is non-replicative in bacteria of the genera *Escherichia.* In another preferred embodiment, said oriR is non-replicative in bacteria of the genera *Klebsiella.* In another preferred embodiment, said oriR is non-replicative in bacteria of the genera *Shigella.*

In another preferred embodiment, said oriR is non-replicative in *Escherichia coli, Klebsiella pneumoniae, Shigella flexneri, Shigella sonnei* or *Shigella dysenteriae.* In another preferred embodiment, said oriR is non-replicative in *Escherichia coli.* In another preferred embodiment, said oriR is non-replicative in *Klebsiella pneumoniae.* In another preferred embodiment, said oriR is non-replicative in *Shigella flexneri.* In another preferred embodiment, said oriR is non-replicative in *Shigella sonnei.* In another preferred embodiment, said oriR is non-replicative in *Shigella dysenteriae.*

In another preferred embodiment, said oriR is R6K and said bacterium is a member of the *Enterobacteriaceae.* In another preferred embodiment, said oriR is R6K and said bacterium is selected from the group consisting of the genera *Escherichia, Klebsiella, Shigella, Enterobacter, Yersinia, Salmonella, Serratia, Citrobacter,* and *Proteus,* preferably of the genera *Escherichia, Klebsiella,* or *Shigella.*

The vector of the invention comprises a restriction endonuclease gene, a recognition site of a restriction endonuclease encoded by said restriction endonuclease gene, and a second regulatory sequence, wherein said second regulatory sequence is operatively linked to said restriction endonuclease gene.

A restriction endonuclease gene is a nucleic acid sequence encoding a restriction endonuclease. The term "restriction endonuclease", also called restriction enzyme, as used herein refers to an enzyme that cleaves DNA molecules into fragments at or near specific recognition sites, called restriction sites, within the DNA molecule. To cut DNA, the restriction enzyme makes two incisions, one through each sugar-phosphate backbone (i.e. each strand) of the DNA double helix. Restriction enzymes are commonly classified by their structure and restriction site. In a preferred embodiment, the restriction enzyme encoded on the vector of the invention cuts the DNA substrate at its recognition site, or recognition and cleavage sites are separate from one another. Restriction enzymes encoded on the vector of the invention include and are preferably restriction enzymes of type I to V, artificial restriction enzymes and meganucleases.

In a preferred embodiment, said restriction endonuclease gene encodes a meganuclease. A meganuclease is an endodeoxyribonuclease characterized by a large recognition site. In a preferred embodiment, said meganuclease has a dsDNA restriction site of 12 or more than base pairs. More preferred restriction endonucleases are meganucleases with a dsDNA restriction site of 12-40 base pairs.

Meganucleases provide the advantage to be very specific restriction enzymes. The large restriction sites of meganucleases reduce the risk of unspecific cleavage of the genome.

Meganucleases of the invention are from bacteria, phages, fungi, yeast, algae or plants. In a preferred embodiment of the invention, said meganuclease encoded by the restriction endonuclease gene of the vector is a bacterial meganuclease.

If the vector includes a meganuclease gene then it is selected such that the bacterium selected for genetic manipulation does not carry the target DNA sequence of this endonuclease in its genome. Otherwise the counter selection would lead to unspecific genome degradation and cell death.

In another preferred embodiment, said meganuclease is a homing endonuclease, preferably a bacterial homing endonuclease. In another preferred embodiment, said homing endonuclease is an intron endonuclease or an intein endonuclease.

In a further preferred embodiment, said restriction endonuclease gene encodes a homing endonuclease, wherein said homing endonuclease is selected from the LAGLIDADG family, GIY-YIG family, HNH family, His-Cys box family, and PD-(D/E)XK family.

In a further preferred embodiment, said homing endonuclease is selected from wild-type homing endonucleases, engineered homing endonucleases, chimeric homing endonucleases, putative homing endonucleases and pseudo homing endonucleases.

Preferably said homing endonuclease of the invention is selected from the group consisting of I-AabMI, AI-AniI, I-CeuI, I-CkaMI, I-CpaMI, I-CraMI, I-CreI, I-DmoI, I-GpeMI, I-GpiI, I-GzeI, GI-GzeII, I-HjeMI, I-LtrI, I-LtrWI, I-MpeMI, I-MsoI, I-OnuI, I-PanMI, I-SceI, I-SmaMI, I-SscMI, I-Vdi141I, PI-SceI, I-CreI (m), I-MsoI (m), I-OnuI (E2), I-AniI / I-OnuI, I-DmoI / I-CreI, I-GpiI / I-OnuI, I-GzeI / I-PanMI, I-LtrI / I-PanMI, I-OnuI / I-LtrI, I-AaeMIP, I-ApaMIP, I-GzeMIIIP, I-NcrMIP, I-OsoMIIP, I-OsoMIP, I-PanMIIIP, I-PanMIIP, I-ScuMIIIP, I-ScuMIIP, I-ScuMIP, and I-ScuMIVP.

Preferably said homing endonuclease is a wild-type homing endonuclease selected from the group consisting of I-AabMI, AI-AniI, I-CeuI, I-CkaMI, I-CpaMI, I-CraMI, I-CreI, I-DmoI, I-GpeMI, I-GpiI, I-GzeI, GI-GzeII, I-HjeMI, I-LtrI, I-LtrWI, I-MpeMI, I-MsoI, I-OnuI, I-PanMI, I-SceI, I-SmaMI, I-SscMI, I-Vdi141I, and PI-SceI. Preferably said homing endonuclease is an engineered homing endonuclease selected from the group consisting of I-Crel (m), I-MsoI (m), and I-OnuI (E2). Preferably said homing endonuclease is a chimeric homing endonuclease selected from the group consisting of I-AniI / I-OnuI, I-DmoI / I-CreI, I-GpiI / I-OnuI, I-GzeI / I-PanMI, I-LtrI / I-PanMI, and I-OnuI / I-LtrI. Preferably said homing endonuclease is a putative and/or pseudo homing endonuclease selected from the group consisting of I-AaeMIP, I-ApaMIP, I-GzeMIIIP, I-NcrMIP, I-OsoMIIP, I-OsoMIP, I-PanMIIIP, I-PanMIIP, I-ScuMIIIP, I-ScuMIIP, I-ScuMIP, and I-ScuMIVP.

In another preferred embodiment, the vector according to the invention further comprises a nucleotide sequence consisting of
(i) a first nucleotide sequence, and
(ii) a second nucleotide sequence,
   wherein the first nucleotide sequence is at least 80% identical to an upstream flanking DNA sequence which flanks up-stream a DNA sequence in the bacterial genome, and
   wherein the second nucleotide sequence is at least 80% identical to a downstream flanking DNA sequence which flanks down-stream said DNA sequence in the bacterial genome, and
(iii) optionally, a third nucleotide sequence, wherein in the vector said third nucleotide sequence is at one end flanked by said first nucleotide sequence and at the other end flanked by said second nucleotide sequence; and
wherein said first nucleotide sequence adjoins said second nucleotide sequence in said vector if the optional third nucleotide sequence is not present.

In another preferred embodiment, the vector according to the invention further comprises a nucleotide sequence consisting of
(i) a first nucleotide sequence, and
(ii) a second nucleotide sequence,
wherein the first nucleotide sequence is at least 80% identical to an up-stream flanking DNA sequence which flanks upstream a target DNA sequence in the bacterial genome, and
wherein the second nucleotide sequence is at least 80% identical to a down-stream flanking DNA sequence which flanks downstream said target DNA sequence in the bacterial genome, wherein the first nucleotide sequence adjoins the second nucleotide sequence in the vector.

In a preferred embodiment, the first nucleotide sequence directly adjoins the second nucleotide sequence in the vector. In a preferred embodiment, said up-stream flanking DNA sequence directly flanks upstream said target DNA sequence in the bacterial genome, and said down-stream flanking DNA sequence directly flanks downstream said target DNA sequence in the bacterial genome.

In another preferred embodiment, the vector according to the invention further comprises a nucleotide sequence consisting of
(i) a first nucleotide sequence,
(ii) a second nucleotide sequence, and
(iii) a third nucleotide sequence,
wherein in the vector said third nucleotide sequence is at one end flanked by said first nucleotide sequence and at the other end flanked by said second nucleotide sequence, and wherein the first nucleotide sequence is at least 80% identical to an up-stream flanking DNA sequence in the bacterial genome, and the second nucleotide sequence is at least 80% identical to a down-stream flanking DNA sequence in the bacterial genome, and said upstream and said downstream flanking DNA sequences either directly flank each other in the bacterial genome or said upstream and said downstream flanking DNA sequences flank a DNA sequence located between the upstream and downstream flanking DNA sequence in the bacterial genome. Then said upstream flanking DNA sequences flanks upstream a DNA sequence in the bacterial genome and said downstream flanking DNA sequence flanks downstream the same DNA sequence in the bacterial genome.

In a preferred embodiment, the first nucleotide sequence directly adjoins the second nucleotide sequence in the vector. In a preferred embodiment, said up-stream flanking DNA sequence directly flanks upstream said target DNA sequence in the bacterial genome, and said down-stream flanking DNA sequence directly flanks downstream said target DNA sequence in the bacterial genome.

Said first nucleotide sequence is capable of homologous recombination with said up-stream flanking DNA sequence in the bacterial genome, said second nucleotide sequence is capable of homologous recombination with said down-stream flanking DNA sequence in the bacterial genome.

The sequence identity, expressed in terms of %, is herein defined as the amount of nucleotides which match exactly between two sequences, wherein the shorter sequence is compared with the longer sequence, if applicable.

In a preferred embodiment, the first nucleotide sequence of the vector of the invention is at least 85% identical to the upstream flanking DNA sequence flanking upstream a DNA sequence in the bacterial genome, and the second nucleotide sequence is at least 85% identical to the downstream flanking DNA sequence flanking downstream a DNA sequence in the bacterial genome. In another preferred embodiment, the first nucleotide sequence of the vector of the invention is at least 90% identical to the up-stream flanking DNA sequence, and the second nucleotide sequence is at least 90% identical to the downstream flanking DNA sequence. In another preferred embodiment, the first nucleotide sequence of the vector of the invention is at least 95% identical to the up-stream flanking DNA sequence, and the second nucleotide sequence is at least 95% identical to the down-stream flanking DNA sequence. In another preferred embodiment, the first nucleotide sequence of the vector of the invention is at least 98% identical to the up-stream flanking DNA sequence, and the second nucleotide sequence is at least 98% identical to the down-stream flanking DNA sequence. In another preferred embodiment, the first nucleotide sequence of the vector of the invention is at least 99% identical to the up-stream flanking DNA sequence, and the second nucleotide sequence is at least 99% identical to down-stream flanking DNA sequence. In another preferred embodiment, the first nucleotide sequence of the vector of the invention is at least 100% identical to the up-stream flanking DNA sequence, and the second nucleotide sequence is at least 100% identical to down-stream flanking DNA sequence. In another preferred embodiment, the first nucleotide sequence of the vector of the invention has the same sequence as the up-stream flanking DNA sequence, and the second nucleotide sequence has the same sequence as the down-stream flanking DNA sequence.

In a preferred embodiment, said DNA sequence in the bacterial genome flanked up-stream and down-stream by said flanking DNA sequences is a target DNA sequence. The term "target DNA sequence" includes and preferably refers to a certain gene (i.e. a target gene), part of a gene, more than one gene (e.g. a group of interconnected genes), a DNA stretch (i.e. a DNA sequence), a regulatory sequence or combinations thereof, e.g. a bacterial target gene and its regulatory sequence(s). The term "target DNA sequence" in the bacterial genome refers to a target DNA sequence located within the bacterial genome. Preferably, said target DNA sequence or said flanked DNA sequence is a bacterial DNA sequence. In a preferred embodiment, said target DNA sequence or said flanked DNA sequence in the bacterial genome can be a sequence of zero nucleotides (nts), but has preferably a length of a few nts up to several thousand nts.

In a preferred embodiment, said target DNA sequence in the bacterial genome flanked up-stream and down-stream by said flanking DNA sequences is a target DNA sequence to be knocked-out of the genome of the bacterium to be manipulated, i.e. said target DNA sequence is a knock-out sequence.

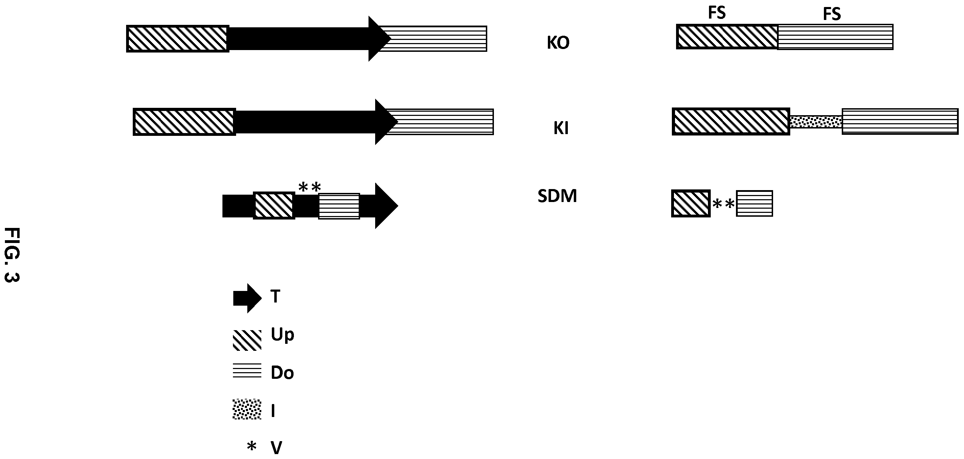

In a further preferred embodiment, said target DNA sequence is a knock-out sequence, and said third nucleotide sequence is not included in the vector of the invention. For knocking-out, but not knocking-in a nucleotide sequence, the third nucleotide sequence is not present in the vector. Then said first nucleotide sequence adjoins (i.e. is located directly adjacent to) the second nucleotide sequence (Fig. 3, left part).

In a preferred embodiment, the upstream flanking nucleotide sequence is located directly upstream (5') to the target DNA sequence, and the downstream flanking nucleotide sequence is located directly downstream (3') to the target DNA in the bacterial genome.

For knocking-in a nucleotide sequence, the third nucleotide sequence has to be included in the vector of the invention: The third nucleotide sequence is then located between said first and said second nucleotide sequence and directly adjoins said first and said second nucleotide sequence (Fig. 3, middle and right part). Thus, in a preferred embodiment, said third nucleotide sequence is included in the vector of the invention. Preferably, said third nucleotide sequence is a naturally occurring gene, a modified gene or part of a modified gene, more than one gene (e.g. a group of interconnected genes), a DNA stretch (i.e. a DNA sequence), a regulatory sequence, or combinations thereof, e.g. a gene and its regulatory sequence(s). In a preferred embodiment, said third nucleotide sequence is a sequence to be knocked-in into the bacterial genome, i.e. said third nucleotide sequence is a knock-in sequence.

Preferably, said third nucleotide sequence is included in the vector, is a knock-in sequence and said up-stream and said down-stream flanking DNA sequences directly flank each other in the bacterial genome, i.e. the up-stream flanking DNA sequence directly adjoins the down-stream flanking DNA sequence in the bacterial genome. In this embodiment the third nucleotide sequence included in the vector is knocked-in into the bacterial genome to be manipulated, and no gene is knocked out.

In a preferred embodiment, said third nucleotide sequence is at one end directly flanked by said first nucleotide sequence and at the other end directly flanked by said second nucleotide sequence.

For knocking-out and knocking-in a nucleotide sequence, the third nucleotide sequence is present in the vector, and said up-stream and down-stream flanking sequences flank the knock-out sequence in the bacterial genome. In a preferred embodiment, said DNA sequence in the bacterial genome flanked up-stream and down-stream by said flanking DNA sequences is a target DNA sequence to be knock-out of the genome of the bacterium to be manipulated (knock-out sequence) and said third nucleotide sequence included in the vector is a knock-in sequence.

In a preferred embodiment, said third nucleotide sequence is modified by site-directed mutagenesis, e.g., compared to a naturally occurring nucleotide sequence. Preferably, said third nucleotide sequence is a modification of a target DNA sequence of the bacterial genome. In a preferred embodiment, said third nucleotide sequence is 100%, 99%, 95%, 90% or 80% identical to said target DNA sequence of the bacterial genome. Modifications are preferably introduced into the target DNA sequence by exchanging, deleting or adding one or more nucleotides, codons or DNA stretches.

In a preferred embodiment, a restriction site is included in the third nucleotide sequence. This has the advantage that the restriction site can be introduced into the bacterial genome via the vector of the invention and during PCR screen a restriction digestion is possible in order to cleave the PCR product at the introduced site. This may be used as a proof for a successful genetic manipulation (e.g. for knock-in or site directed mutagenesis).

In another preferred embodiment, the non-antibiotic selection marker gene cassette is selected from the group consisting of a heavy metal resistance gene, triclosan resistance gene, glyphosate resistance gene, bialaphos resistance gene, and phosphinothricin resistance gene.

| **Selection agent** | **Gene encoding the resistance** |
|---|---|
| glyphosate | Gat (glyphosate N-acetyltransferase) |
| bialaphos and its degradation product, phosphinothricin (PPT) | Bar (phosphinotricin acetylase) |
| inorganic or organic mercury compounds | mer genes (merAB, merA) |
| arsenite and arsenate | ars genes (arsABC, arsAB) |
| tellurite | tellurite resistance genes (tpm, kilA/telA/telB, terC, terB, tehAB operon, kilA operon (klaA klaB telB), terWZA-F genes) |

In another preferred embodiment, the non-antibiotic selection marker gene is a heavy metal resistance gene. In another more preferred embodiment, the non-antibiotic selection marker gene is a heavy metal resistance gene selected from the group consisting of an arsenic resistance gene, tellurite resistance gene, and mercury resistance gene.

In another again more preferred embodiment, said non-antibiotic selection marker gene is a tellurite resistance gene. In an even more preferred embodiment, said non-antibiotic selection marker gene is the tellurite resistance gene tpm encoding thiopurine S-methyltransferase. Most preferred is the tpm gene encoding thiopurine S-methyltransferase of Acinetobacter baylyi, preferably at locus tag ACIAD2922.

In a preferred embodiment, the tpm gene has the following sequence (SEQ ID NO: 1; tpm (5'-3'), 654 nucleotides):

ATGCAACATGAGTTCTGGCATCAACGCTGGCAGGAAAACAGAATCGGTTTT
CATCAATTTACACCGAGTCCACTGTTAGTCGATTATTTAACGAACTTGGCTTAAAA
ACCTCTGCCCGTATCTTTGTTCCATTGAGTGGTAAAACACTGGATATTAGTTGGTTA
TTACAACAAGGCTACCATGTTGTGGCAATTGAGCTTAGCCAAATCGCTGTAACATC
ATTGATTGAGCAATTAGTTGAAGACTTCGATATACAATTTGAGTCTTCCGAAAAGAA
TAATCTGATCCATTACCATCACCCACAAATTGATATTTTTGTCGGTGATTTTTTTGAT
CTAAGCAAAGAACAATTAGGACAGGTCGATGCAATTTTTGATCGTGCAGCATTGAT
TGCTTTACCAGACGACATACGCCAAGACTATGTTCAACACTTGATCGAGATCAGTG
GTGCTGCATCTCAGTTTTTAATTAGCTACCAATACGATGCAGGCAGTCATGAAGGA
CCTCCTTTTTCGGTCAATGCAGAAGAAATTAAACAACTTTATGCTGAGGCTTATGA
CATCCGACTTTTGAAGGAGCAACTTGTCGATGCATCTCAAAACAAAGGAAATCAC
CCCAAGAGCACACTATGGATACTCACTGCCAAGTTCTGA

The protein sequence encoded by tpm is preferably the following (SEQ ID NO: 2; 217 amino acids, molecular weight 24987.7 Da):

MQHEFWHQRWQENRIGFHQFTPSPLLVDYFNELGLKTSARIFVPLSGKTLDIS
WLLQQGYHVVAIELSQIAVTSLIEQLVEDFDIQFESSEKNNLIHYHHPQIDIFVGDFFDL
SKEQLGQVDAIFDRAALIALPDDIRQDYVQHLIEISGAASQFLISYQYDAGSHEGPPFS
VNAEEIKQLYAEAYDIRLLKEQLVDASQNKGNHPKSTLWILTAKF

In another again more preferred embodiment, said non-antibiotic selection marker gene is a tellurite resistance gene and said first regulatory sequence is a constitutive bacterial promoter. In another again more preferred embodiment, said non-antibiotic selection marker gene is tpm, and said first regulatory sequence is a constitutive bacterial promoter.

In another preferred embodiment, said non-antibiotic selection marker gene cassette has a length of less than 2000 bp, preferably less than 1000 bp, more preferably less than 700 bp, again more preferably about 650 bp.

In another preferred embodiment, the vector of the invention has a length of less than 5000 bp, preferably less than 4500 bp, more preferably less than about 4000 bp

In another preferred embodiment, in the vector of the invention, the (a) the non-antibiotic selection marker gene cassette, (b) the origin of replication, (c) the restriction endonuclease gene, the recognition site, and the second regulatory sequence and (d) the oriT have together a length of less than 5000 bp, preferably less than 4500 bp, more preferably less than about 4000 bp, again more preferably less than 4200 bp.

In a further preferred embodiment, said first nucleotide sequence and said second nucleotide sequence together have a length of 700 bp or less, preferably 500 bp or less, more preferably 400 bp or less, again more preferably 300 bp or less.

In a further preferred embodiment, said restriction endonuclease gene encodes a homing endonuclease, wherein said homing endonuclease is a member of the family of LAGLIDADG homing endonucleases. In a further preferred embodiment, said member of the LAGLIDADG family possess either one or two copies of the conserved LAGLIDADG motif. In another preferred embodiment, said member of the LAGLIDADG family possess one copy of the LAGLIDADG motif, such as I-CreI. In a further preferred embodiment, said member of the LAGLIDADG family possess two copies of the LAGLIDADG motif, such as I-SceI. In a preferred embodiment, said member of the LAGLIDADG family is selected from the group consisting of I-Crel, I-MsoI, I-DmoI, I-AniI, I-SceI, PI-SceI, and PI-PfuI. In another preferred embodiment, said member of the family of LAGLIDADG homing endonucleases is I-SceI.

In another preferred embodiment, said restriction endonuclease gene encodes the LAGLIDADG homing endonucleases I-SceI. Preferably said I-SceI has the following sequence (SEQ ID NO: 3; 702 nucleotides, I-SceI (5'-3'), encoding 233 amino acids):

ATGCATCAAAAAAACCAGGTAATGAACCTGGGTCCGAACTCTAAACTGCTG
AAAGAATACAAATCCCAGCTGATCGAACTGAACATCGAACAGTTCGAAGCAGGTA
TCGGTCTGATCCTGGGTGATGCTTACATCCGTTCTCGTGATGAAGGTAAAACCTACT
GTATGCAGTTCGAGTGGAAAAACAAAGCATACATGGACCACGTATGTCTGCTGTAC
GATCAGTGGGTACTGTCCCCGCCGCACAAAAAACAACGTGTTAACCACCTGGGTA
ACCTGGTAATCACCTGGGGCGCCCAGACTTTCAAACACCAAGCTTTCAACAAACT
GGCTAACCTGTTCATCGTTAACAACAAAAAAACCATCCCGAACAACCTGGTTGAA
AACTACCTGACCCCGATGTCTCTGGCATACTGGTTCATGGATGATGGTGGTAAATG
GGATTACAACAAAAACTCTACCAACAAATCGATCGTACTGAACACCCAGTCTTTCA
CTTTCGAAGAAGTAGAATACCTGGTTAAGGGTCTGCGTAACAAATTCCAACTGAAC
TGTTACGTAAAAATCAACAAAAACAAACCGATCATCTACATCGATTCTATGTCTTAC
CTGATCTTCTACAACCTGATCAAACCGTACCTGATCCCGCAGATGATGTACAAACT
GCCGAACACTATCTCCTCCGAAACTTTCCTGAAATAA

The protein sequence encoded by I-SceI is preferably the following (SEQ ID NO: 4; 233 amino acids, molecular 27463.5 Da):

MHQKNQVMNLGPNSKLLKEYKSQLIELNIEQFEAGIGLILGDAYIRSRDEGKT
YCMQFEWKNKAYMDHVCLLYDQWVLSPPHKKQRVNHLGNLVITWGAQTFKHQAF

NKLANLFIVNNKKTIPNNLVENYLTPMSLAYWFMDDGGKWDYNKNSTNKSIVLNTQ
SFTFEEVEYLVKGLRNKFQLNCYVKINKNKPIIYIDSMSYLIFYNLIKPYLIPQMMYKL
PNTISSETFLK

I-SceI-meganuclease has the advantage that its DNA recognition site is very specific and composed of 18 distinct nucleotides. Thus, the likelihood that a similar sequence is encoded in the genome of the bacterium to be manipulated is very small.

In a preferred embodiment, said restriction endonuclease gene encodes a LAGLIDADG homing endonuclease, and said non-antibiotic selection marker gene is tpm. In another preferred embodiment, said restriction endonuclease gene encodes the LAGLIDADG homing endonucleases I-SceI, and said non-antibiotic selection marker gene is tpm. In another preferred embodiment, said restriction endonuclease gene encodes the LAGLIDADG homing endonucleases I-SceI of SEQ ID NO: 4, and said non-antibiotic selection marker gene is tpm.

In another preferred embodiment, said restriction endonuclease gene encodes the LAGLIDADG homing endonucleases I-SceI, said non-antibiotic selection marker gene is tpm, said first regulatory sequence is a constitutive bacterial promoter, and said second regulatory sequence is an inducible bacterial promoter.

In another preferred embodiment, said bacterium to be manipulated is a member of the *Enterobacteriaceae.* In another preferred embodiment, said bacterium to be manipulated is selected from the group consisting of the genera *Escherichia, Klebsiella, Shigella, Enterobacter, Yersinia, Salmonella, Serratia, Citrobacter,* and *Proteus. Yersinia* includes or preferably is *Yersinia pestis.* In another preferred embodiment, said bacterium to be manipulated is of the genera *Escherichia, Klebsiella,* or *Shigella.* In another preferred embodiment, said bacterium to be manipulated is of the genera *Escherichia.* In another preferred embodiment, said bacterium to be manipulated is of the genera *Klebsiella.* In another preferred embodiment, said bacterium to be manipulated is of the genera *Shigella.* In another preferred embodiment, said bacterium to be manipulated is *Escherichia coli, Klebsiella pneumoniae, Shigella flexneri, Shigella sonnei* or *Shigella dysenteriae.* In another preferred embodiment, said bacterium to be manipulated is *Escherichia coli.* In another preferred embodiment, said bacterium to be manipulated is *Klebsiella pneumoniae.* In another preferred embodiment, said bacterium to be manipulated is *Shigella flexneri.* In another preferred embodiment, said bacterium to be manipulated is *Shigella sonnei.* In another preferred embodiment, said bacterium to be manipulated is *Shigella dysenteriae.*

Preferably, said bacterium is from a strain that is resistant against antibiotics. Again more preferably, said bacterium is from a strain that is resistant against multiple antibiotics.

In a preferred embodiment, said second regulatory sequence is inducible. In another preferred embodiment, said second regulatory sequence is a chemically or physically inducible promoter. In another preferred embodiment, said second regulatory sequence is inducible by the presence or absence of a compound selected from the group consisting of alcohols, antibiotics, steroids, metals, and saccharides. Said saccarides are preferably monosaccharides or disaccharides.

Said antibiotic is preferably a tetracycline or a tetracycline variant. Said second regulatory sequence is preferably a bacterial promoter inducible by tetracycline or a tetracycline variant selected from the group consisting of anhydrotetracycline, minocycline, metacycline, sanocycline, demeclocycline, chloro-tetracycline, oxytetracycline, doxycycline, and tigecycline. In another preferred embodiment, said second regulatory sequence is inducible by lactose or isopropyl-β-D-1-thiogalactopyranoside (IPTG) or arabinose.

In another preferred embodiment, said restriction endonuclease gene encodes the LAGLIDADG homing endonucleases I-SceI, said non-antibiotic selection marker gene is tpm, said first regulatory sequence is a constitutive bacterial promoter, said second regulatory sequence is an inducible bacterial promoter, and said oriR is not capable of inducing replication in *Enterobacteriaceae.* In another preferred embodiment, said restriction endonuclease gene encodes the LAGLIDADG homing endonucleases I-SceI, said non-antibiotic selection marker gene is tpm, said first regulatory sequence is a constitutive bacterial promoter, said second regulatory sequence is an inducible bacterial promoter, and said oriR is not capable of inducing replication in *Enterobacteriaceae* selected from the group consisting of the genera *Escherichia, Klebsiella, Shigella, Enterobacter, Yersinia, Salmonella, Serratia, Citrobacter,* and *Proteus.* In another preferred embodiment, said restriction endonuclease gene encodes the LAGLIDADG homing endonucleases I-SceI, said non-antibiotic selection marker gene is tpm, said first regulatory sequence is a constitutive bacterial promoter, said second regulatory sequence is an inducible bacterial promoter, and said oriR is not capable of inducing replication in *Escherichia, Klebsiella,* or *Shigella.* In another preferred embodiment, said restriction endonuclease gene encodes the LAGLIDADG homing endonucleases I-SceI, said non-antibiotic selection marker gene is tpm, said first regulatory sequence is a constitutive bacterial promoter, said second regulatory sequence is an bacterial promoter inducible by tetracycline or a tetracycline variant selected from the group consisting of anhydrotetracycline, minocycline, metacycline, sanocycline, demeclocycline, chloro-tetracycline, oxytetracycline, doxycycline, and tigecycline, by lactose, isopropyl-β-D-1-thiogalactopyranoside (IPTG) or arabinose; and said oriR is not capable of inducing replication in *Escherichia, Klebsiella,* or *Shigella.*

Preferably said vector of the invention further comprises a another selection marker gene cassette comprising a further marker gene and a third regulatory sequence operatively linked to said further marker gene. Preferably said further marker gene is an antibiotic marker gene. In preferred embodiment, said further marker gene is a resistance gene or gene cassette of kanamycin, neomycin, or combination of both (kanR/neo).

In another preferred embodiment, said restriction endonuclease gene encodes the LAGLIDADG homing endonucleases I-SceI, said non-antibiotic selection marker gene is tpm, said first regulatory sequence is a constitutive bacterial promoter, said second regulatory sequence is an inducible bacterial promoter, and said oriR is not capable of inducing replication in *Escherichia, Klebsiella,* or *Shigella,* and said vector of the invention further comprises a second selection marker gene cassette comprising an antibiotic marker gene and a third regulatory sequence operatively linked to said antibiotic marker gene.

In a preferred embodiment, said vector of the invention comprises a multi-cloning site.

In a further aspect, the invention relates to a bacterium comprising the vector of the invention.

Said bacterium (also called bacterial host cell) is in a preferred embodiment a member of the *Enterobacteriaceae.* In another preferred embodiment, said bacterium is selected from the group consisting of the genera *Escherichia, Klebsiella, Shigella, Enterobacter, Yersinia, Salmonella, Serratia, Citrobacter,* and *Proteus.* Yersinia includes or preferably is Yersinia pestis. In another preferred embodiment, said bacterium is of the genera *Escherichia, Klebsiella,* or *Shigella.* In another preferred embodiment, said bacterium is of the genera *Escherichia.* In another preferred embodiment, said bacterium is of the genera *Klebsiella.* In another preferred embodiment, said bacterium is of the genera *Shigella.*

In another preferred embodiment, said bacterium is *Escherichia coli, Klebsiella pneumoniae, Shigella flexneri, Shigella sonnei* or *Shigella dysenteriae.* In another preferred embodiment, said bacterium is *Escherichia coli.* In another preferred embodiment, said bacterium is *Klebsiella pneumoniae.* In another preferred embodiment, said bacterium is *Shigella flexneri.* In another preferred embodiment, said bacterium is *Shigella sonnei.* In another preferred embodiment, said bacterium is *Shigella dysenteriae.*

Preferably, said bacterium is from a strain that is resistant against antibiotics. Again more preferably, said bacterium is from a strain that is resistant against multiple antibiotics.

In a further aspect, the invention relates to a method for genetic manipulation of bacteria comprising the steps of:
(a) transferring the vector of the invention, comprising said first and said second nucleotide sequences or comprising said first, said second and said third nucleotide sequence, into bacterial host cells,
(b) culturing said bacterial host cells in a first medium,
(c) selecting from the cultured bacterial host cells of step (b), bacterial host cells having the vector integrated into their genome,
(d) culturing the selected bacterial host cells of step (c) in a second medium, and
(e) counter-selecting from the cultured bacterial host cells of step (d), bacterial host cells in which said target DNA sequence is eliminated from the bacterial genome (i.e. disintegrated from the bacterial genome), and/or bacterial host cells in which said third nucleotide sequence is integrated into the bacterial genome.

Said bacterial host cells selected in step c), having the vector integrated into their genome, either express or do not express said non-antibiotic selection marker gene of the vector of the invention.

In the counter-selected bacterial host cells in which said target DNA sequence is eliminated from the bacterial genome, the target DNA sequence is knocked out. In the counter-selected bacterial host cells bacterial host cells, in which said third nucleotide sequence is integrated into the bacterial genome, the target DNA sequence is knocked-in into the bacterial genome.

The selection of bacterial host cells having the vector integrated into their bacterial genome in step (c) is preferably based on the expression of said non-antibiotic selection marker gene of the vector. Thus, in a preferred embodiment, bacterial host cells having the vector integrated into their bacterial genome are selected by detecting expression of said non-antibiotic selection marker gene of the vector. In a preferred embodiment, said non-antibiotic selection marker gene is selected from the group consisting of a heavy metal resistance gene, triclosan resistance gene, glyphosate resistance gene, bialaphos resistance gene, and phosphinothricin resistance gene. More preferably, said non-antibiotic selection marker gene is a heavy metal resistance gene selected from the group consisting of an arsenic resistance gene, tellurite resistance gene, and mercury resistance gene. Again more preferably, said non-antibiotic selection marker gene is a tellurite resistance gene, and again more preferably thiopurine S-methyltransferase (tpm).

In a preferred embodiment, integration of the vector of the invention into the genome of the bacterial host cells is confirmed by PCR.

In another preferred embodiment of the method of the invention, said non-antibiotic selection marker gene of the vector of the invention is a tellurite resistance gene, and said medium of step (b) contains more than 100 µg/ml tellurite. In another preferred embodiment of the method of the invention, said non-antibiotic selection marker gene of the vector of the invention is thiopurine S-methyltransferase (tpm), and said medium of step (b) contains more than 100 µg/ml tellurite.

In another preferred embodiment of the method of the invention, said non-antibiotic selection marker gene of the vector of the invention is a tellurite resistance gene, and said medium of step (b) contains 200 µg/ml tellurite or more, preferably of 300 ug/ml tellurite or more, and further preferably of 400 µg/ml tellurite or more. In another preferred embodiment of the method of the invention, said non-antibiotic selection marker gene of the vector of the invention is thiopurine S-methyltransferase (tpm), and said medium of step (b) contains 200 µg/ml tellurite or more, preferably of 300 µg/ml tellurite or more, and further preferably of 400 µg/ml tellurite or more.

In another preferred embodiment of the method of the invention, said second regulatory sequence of the vector of the invention is a bacterial promoter inducible by an inducer, and said medium of step (d) contains said inducer. In a preferred embodiment said inducer is tetracycline or a tetracycline variant selected from the group consisting of anhydrotetracycline, minocycline, metacycline, sanocycline, demeclocycline, chloro-tetracycline, oxytetracycline, doxycycline, and tigecycline.

In the vector of the invention, said second regulatory sequence is operatively linked to said restriction endonuclease gene, which is preferably a member of the family of LAGLIDADG homing endonucleases, more preferably said member of the family of LAGLIDADG homing endonucleases is I-SceI.

The counter-selection of bacterial host cells having the target DNA sequence eliminated from the bacterial genome or having the third nucleotide sequence integrated into the bacterial genome in step (e) is preferably based on the activity of the restriction endonuclease encoded by said restriction endonuclease gene. Thus, in a preferred embodiment, bacterial host cells having the target DNA sequence eliminated from their bacterial genome or having the third nucleotide sequence integrated into their bacterial genome are selected by detecting activity of the restriction endonuclease encoded by said restriction endonuclease gene of the vector. In a preferred embodiment, said restriction endonuclease is a member of the family of LAGLIDADG homing endonucleases. More preferably, said restriction endonuclease is I-SceI.

In a preferred embodiment, elimination of said target DNA sequence from the bacterial genome or integration of said third nucleotide sequence into the bacterial genome of the bacterial host cells is confirmed by PCR.

In a particularly preferred embodiment, said second regulatory sequence of the vector of the invention is a bacterial promoter inducible by an inducer which is tetracycline or a tetracycline variant selected from the group consisting of anhydrotetracycline, minocycline, metacycline, sanocycline, demeclocycline, chloro-tetracycline, oxytetracycline, doxycycline, and tigecycline, wherein said inducer is included in the medium of step (d); and said second regulatory sequence is operatively linked to said restriction endonuclease gene, which is a member of the family of LAGLIDADG homing endonucleases, preferably said member of the family of LAGLIDADG homing endonucleases is I-SceI.

In another preferred embodiment of the method of the invention, said non-antibiotic selection marker gene of the vector of the invention is thiopurine S-methyltransferase (tpm), and said medium of step (b) contains more than 100 µg/ml tellurite; said second regulatory sequence of the vector of the invention is a bacterial promoter inducible by an inducer, and said medium of step (d) contains said inducer selected from the group consisting of tetracycline, anhydrotetracycline, minocycline, metacycline, sanocycline, demeclocycline, chloro-tetracycline, oxytetracycline, doxycycline, and tigecycline.

In a preferred embodiment of the method of the invention, said bacterial host cell is a member of the *Enterobacteriaceae.* In another preferred embodiment, said bacterial host cell is selected from the group consisting of the genera *Escherichia, Klebsiella, Shigella, Enterobacter, Yersinia, Salmonella, Serratia, Citrobacter,* and *Proteus. Yersinia* includes or preferably is *Yersinia pestis.* In another preferred embodiment, said bacterial host cell is of the genera *Escherichia, Klebsiella, or Shigella.* In another preferred embodiment, said bacterial host cell is of the genera *Escherichia.* In another preferred embodiment, said bacterial host cell is of the genera *Klebsiella.* In another preferred embodiment, said bacterial host cell is of the genera *Shigella.* In another preferred embodiment, said bacterial host cell is *Escherichia coli, Klebsiella pneumoniae,* or *Shigella flexneri.* In another preferred embodiment, said bacterial host cell is *Escherichia coli, Klebsiella pneumoniae, Shigella flexneri, Shigella sonnei* or *Shigella dysenteriae.* In another preferred embodiment, said bacterial host cell is *Klebsiella pneumoniae.* In another preferred embodiment, said bacterial host cell is *Shigella flexneri.* In another preferred embodiment, said bacterium is *Shigella sonnei.* In another preferred embodiment, said bacterium is *Shigella dysenteriae.* Preferably, said bacterial host cells are cultured at 30°C.

Preferably, said bacterial host cells used in the method of the invention are from a strain that is resistant against antibiotics. Again more preferably, said bacterial host cells are from a strain that is resistant against multiple antibiotics.

In another preferred embodiment, said bacterial host cells selected in step (c) have the vector integrated into their genome via targeted integration. Preferably, no external recombinases are added in the method according to the invention. In a preferred embodiment, endogenous recombinases of the bacterial host cells are used.

Preferably, said vector of the invention is transferred into the bacterial host cells via conjugation. Thus, in a preferred embodiment, said bacterial host cells are conjugative.

In another preferred embodiment, said medium of step (b) and/or said medium of step (d) contains a compound selected from the group consisting of a bicyclic compound substituted with at least one hydroxyl group or at least one amino group; a branched fatty acid having a chain length of more than C13; and an unsaturated fatty acid having a chain length of more than C14.

In another preferred embodiment, said medium of step (b) and/or said medium of step (d) contains a bicyclic compound substituted with at least one hydroxyl group or at least one amino group. In another preferred embodiment, said medium of step (b) and/or said medium of step (d) contains a branched fatty acid having a chain length of more than C13. In another preferred embodiment, said medium of step (b) and/or said medium of step (d) contains an unsaturated fatty acid having a chain length of more than C14.

In a preferred embodiment, said bicyclic compound is selected from the group consisting of hydroxy-indole, hydroxy-naphthalene, and amino-naphthalene. In another preferred embodiment, said bicyclic compound is selected from the group consisting of 4-hydroxyindol, 5-hydroxyindol, 6-hydroxyindol, 7-hydroxyindol, 1-naphtol, 2-naphtol, 1,2-dihydroxynaphtalene, 1,3-dihydroxynaphtalene 1,4-dihydroxynaphtalene, 2,3-dihydroxynaphtalene, 1-amino-naphthalene, and 2-amino-naphthalene.

In a further preferred embodiment, said branched fatty acid has a carbon chain length of C14 to C35, preferably C14 to C30, more preferably C14 to C25, again more preferably C14 to C20, and most preferably C15 to C17. In a further preferred embodiment, said branched fatty acid is selected from the group consisting of 12-methyltridecanoic acid (iso-C14:0), 12-methyltetradecanoic acid (anteiso-C15:0), 13-methyltetradecanoic acid (iso-C15:0), 14-methylpentadecanoic acid (iso-C16:0), 14-methylhexa-decanoic acid (anteiso-C17:0), and 15-methylhexadecanoic acid (iso-C17:0).

In a further preferred embodiment, said unsaturated fatty acid has a carbon chain length of C15 to C35, preferably C15 to C30, more preferably C15 to C25, again more preferably C15 to C20, and most preferably C16 to C18. In a further preferred embodiment, said unsaturated fatty acid is selected from the group consisting of 9-hexadecenoic acid (palmitoleic acid, C16:1), cis-9-octadecenoic acid (oleic acid, C18:1), cis-11-octadecenoic acid (vaccenic acid, C18:1), cis-9, and cis-12-octadecadienoic acid (linoleic acid, C18:2).

In a further aspect, the invention relates to a method for selecting bacterial host cells comprising the steps of:
(a) transferring a vector into bacterial host cells, wherein said vector comprises
   - a thiopurine S-methyltransferase (tpm) gene, and
   - a regulatory sequence operatively linked to said tpm gene,
(b) culturing said bacterial host cells in a medium containing more than 100 µg/ml tellurite,
(c) selecting bacterial host cells that survived the tellurite concentration of step (b).

In another preferred embodiment, said medium contains 200 µg/ml tellurite or more, preferably of 300 µg/ml tellurite or more, and further preferably of 400 µg/ml tellurite or more.

In another preferred embodiment, said bacterial host cells are from the genus *Pseudomonas.* Preferably, said bacterial host cells are from the species *Pseudomonas aeruginosa.* More preferably, said bacterial host cells are from a strain that is resistant against antibiotics. Again more preferably, said bacterial host cells are from a strain that is resistant against multiple antibiotics.

Preferably, said vector is transferred into the bacterial host cells via conjugation. Thus, in a preferred embodiment, said bacterial host cells are conjugative. In a preferred embodiment, said vector of the invention comprises an origin of transfer (oriT). The oriT is required for the plasmid transfer via conjugation.

In another preferred embodiment, said bacterial host cells selected in step (c) have the vector integrated into their genome via targeted integration.

In another preferred embodiment, said vector comprising a tpm gene and a regulatory sequence operatively linked to said tpm gene (tpm vector) further comprises an origin of replication, wherein said origin of replication is not capable of inducing replication in said bacterial host cells. In another preferred embodiment, said tpm vector further comprises an origin of replication, wherein said origin of replication is not capable of inducing replication in bacterial host cells from the genus *Pseudomonas.* In a further preferred embodiment, said bacterial host cells from the genus *Pseudomonas* are from the species *Pseudomonas aeruginosa.* In another preferred embodiment, said origin of replication is colE1-ori.

In another preferred embodiment, said tpm vector comprises an origin of transfer (oriT).

In another preferred embodiment, said tpm vector further comprises a nucleotide sequence consisting of
(i) a first nucleotide sequence, and
(ii) a second nucleotide sequence,
   wherein the first nucleotide sequence is at least 80% identical to an upstream flanking DNA sequence flanking upstream a DNA sequence in the bacterial genome, and the second nucleotide sequence is at least 80% identical to a downstream flanking DNA sequence flanking downstream said DNA sequence in the bacterial genome, and
(iii) optionally, a third nucleotide sequence, wherein in the tpm vector said third nucleotide sequence is at one end flanked by said first nucleotide sequence and at the other end flanked by said second nucleotide sequence; and wherein said first nucleotide sequence adjoins said second nucleotide sequence in said tpm vector if the optional third nucleotide sequence is not present.

In another preferred embodiment, said tpm vector further comprises a nucleotide sequence consisting of (i) a first nucleotide sequence, and (ii) a second nucleotide sequence, wherein the first nucleotide sequence is at least 80% identical to an up-stream flanking DNA sequence which flanks upstream a target DNA sequence in the bacterial genome, and wherein the second nucleotide sequence is at least 80% identical to a down-stream flanking DNA sequence which flanks downstream said target DNA sequence in the bacterial genome, wherein the first nucleotide sequence adjoins the second nucleotide sequence in the vector.

In another preferred embodiment, said tpm vector further comprises a nucleotide sequence consisting of (i) a first nucleotide sequence, (ii) a second nucleotide sequence, and (iii) a third nucleotide sequence, wherein in the vector said third nucleotide sequence is at one end flanked by said first nucleotide sequence and at the other end flanked by said second nucleotide sequence, and wherein the first nucleotide sequence is at least 80% identical to an up-stream flanking DNA sequence in the bacterial genome, and the second nucleotide sequence is at least 80% identical to a down-stream flanking DNA sequence in the bacterial genome, and said upstream and said downstream flanking DNA sequences either directly flank each other in the bacterial genome or flank a DNA sequence located between the upstream and downstream flanking DNA sequence in the bacterial genome.

Said first nucleotide sequence is capable of homologous recombination with said up-stream flanking DNA sequence in the bacterial genome, said second nucleotide sequence is capable of homologous recombination with said down-stream flanking DNA sequence in the bacterial genome.

The sequence identity, expressed in terms of %, is herein defined as the amount of nucleotides which match exactly between two sequences, wherein the shorter sequence is compared with the longer sequence, if applicable.

In a preferred embodiment, the first nucleotide sequence of the tpm vector is at least 85% identical to the upstream flanking DNA sequence flanking upstream a DNA sequence in the bacterial genome, and the second nucleotide sequence is at least 85% identical to the downstream flanking DNA sequence flanking downstream a DNA sequence in the bacterial genome. In another preferred embodiment, the first nucleotide sequence of the tpm vector is at least 90% identical to the up-stream flanking DNA sequence, and the second nucleotide sequence is at least 90% identical to the downstream flanking DNA sequence. In another preferred embodiment, the first nucleotide sequence of the tpm vector is at least 95% identical to the up-stream flanking DNA sequence, and the second nucleotide sequence is at least 95% identical to the down-stream flanking DNA sequence. In another preferred embodiment, the first nucleotide sequence of the tpm vector is at least 98% identical to the up-stream flanking DNA sequence, and the second nucleotide sequence is at least 98% identical to the down-stream flanking DNA sequence. In another preferred embodiment, the first nucleotide sequence of the tpm vector is at least 99% identical to the up-stream flanking DNA sequence, and the second nucleotide sequence is at least 99% identical to down-stream flanking DNA sequence. In another preferred embodiment, the first nucleotide sequence of the tpm vector is at least 100% identical to the up-stream flanking DNA sequence, and the second nucleotide sequence is at least 100% identical to down-stream flanking DNA sequence. In another preferred embodiment, the first nucleotide sequence of the tpm vector has the same sequence as the up-stream flanking DNA sequence, and the second nucleotide sequence has the same sequence as the down-stream flanking DNA sequence.

In a preferred embodiment, said DNA sequence in the bacterial genome flanked up-stream and down-stream by said flanking DNA sequences is a target DNA sequence. The term "target DNA sequence" includes and preferably refers to a certain gene (i.e. a target gene), part of a gene, more than one gene (e.g. a group of interconnected genes), a DNA stretch (i.e. a DNA sequence), a regulatory sequence or combinations thereof, e.g. a bacterial target gene and its regulatory sequence(s). The term "target DNA sequence" in the bacterial genome refers to a target DNA sequence located within the bacterial genome. Preferably, said target DNA sequence or said flanked DNA sequence is a bacterial DNA sequence. In a preferred embodiment, said target DNA sequence or said flanked DNA sequence in the bacterial genome can be a sequence of zero nts, but has more preferably a length of a few nts up to several thousand nts.

In a preferred embodiment, said DNA sequence in the bacterial genome flanked up-stream and down-stream by said flanking DNA sequences is a target DNA sequence to be knocked-out of the genome of the bacterium to be manipulated, i.e. said target DNA sequence is a knock-out sequence.

In a further preferred embodiment, said target DNA sequence is a knock-out sequence, and said third nucleotide sequence is not included in the tpm vector. For knocking-out, but not knocking-in a nucleotide sequence, the third nucleotide sequence is not present in the tpm vector. Then said first nucleotide sequence adjoins (i.e. is located directly adjacent to) the second nucleotide sequence (Fig. 3, left part).

In a preferred embodiment, the upstream flanking nucleotide sequence is located directly upstream (5') to the target DNA sequence, and the downstream flanking nucleotide sequence is located directly downstream (3') to the target DNA sequence in the bacterial genome.

For knocking-in a nucleotide sequence, the third nucleotide sequence has to be included in the tpm vector: The third nucleotide sequence is then located between said first and said second nucleotide sequence and directly adjoins said first and said second nucleotide sequence (Fig. 3, middle and right part). Thus, in a preferred embodiment, said third nucleotide sequence is included in the tpm vector. Preferably, said third nucleotide sequence is a naturally occurring gene, a modified gene or part of a modified gene, more than one gene (e.g. a group of interconnected genes), a DNA stretch (i.e. a DNA sequence), a regulatory sequence, or combinations thereof, e.g. a gene and its regulatory sequence(s). In a preferred embodiment, said third nucleotide sequence is a sequence to be knocked-in into the bacterial genome, i.e. said third nucleotide sequence is a knock-in sequence.

In another preferred embodiment, said third nucleic acid included in the tpm vector is a knock-in sequence and said up-stream and said down-stream flanking DNA sequences directly flank each other in the bacterial genome, i.e. the up-stream flanking DNA sequence adjoins the down-stream flanking DNA sequence in the bacterial genome. In this embodiment, the third nucleotide sequence included in the tpm vector is knocked-in into the bacterial genome to be manipulated, and no gene is knocked out.

In a preferred embodiment, said third nucleotide sequence is at one end directly flanked by said first nucleotide sequence and at the other end directly flanked by said second nucleotide sequence.

For knocking-out and knocking-in a nucleotide sequence, the third nucleotide sequence is present in the tpm vector, and said up-stream and down-stream flanking sequences flank the knock-out sequence in the bacterial genome. In a preferred embodiment, said DNA sequence in the bacterial genome flanked up-stream and down-stream by said flanking DNA sequences is a target DNA sequence to be knock-out of the genome of the bacterium to be manipulated (knock-out sequence) and said third nucleotide sequence included in the tpm vector is a knock-in sequence.

In a preferred embodiment, said third nucleotide sequence is modified by site-directed mutagenesis, e.g., compared to a naturally occurring nucleotide sequence. Preferably, said third nucleotide sequence is a modification of a target DNA sequence of the bacterial genome. In a preferred embodiment, said third nucleotide sequence is 100%, 99%, 95%, 90% or 80% identical to said target DNA sequence of the bacterial genome. Modifications are preferably introduced into the target DNA sequence by exchanging, deleting or adding one or more nucleotides, codons or DNA stretches.

In a preferred embodiment, a restriction site is included in the third nucleotide sequence.

In another preferred embodiment, said tpm vector further comprises a second marker gene and a second regulatory sequence, wherein said second regulatory sequence is inducible and operatively linked to said second marker gene. Said second marker gene is for detecting excision of the tpm vector or parts of the tpm vector from the bacterial genome.

In another preferred embodiment, said second marker gene is a thymidine kinase (tdk) gene. Preferably said tdk gene is from *Escherichia coli.* More preferably, said tdk gene has the following sequence (SEQ ID NO: 5; tdk gene (5'-3'), 618 nucleotides):

ATGGCACAGCTATATTTCTACTATTCCGCAATGAATGCGGGTAAGTCTACAGC
ATTGTTGCAATCTTCATACAATTACCAGGAACGCGGCATGCGCACTGTCGTATATAC
GGCAGAAATTGATGATCGCTTTGGTGCCGGGAAAGTCAGTTCGCGTATAGGTTTGT
CATCGCCTGCAAAATTATTTAACCAAAATTCATCATTATTTGATGAGATTCGTGCGG
AACATGAACAGCAGGCAATTCATTGCGTACTGGTTGATGAATGCCAGTTTTTAACC
AGACAACAAGTATATGAATTATCGGAGGTTGTCGATCAACTCGATATACCCGTACTT
TGTTATGGTTTACGTACCGATTTTCGAGGTGAATTATTTATTGGCAGCCAATACTTAC

TGGCATGGTCCGACAAACTGGTTGAATTAAAAACCATCTGTTTTGTGGCCGTAAA
GCAAGCATGGTGCTGCGTCTTGATCAAGCAGGCAGACCTTATAACGAAGGTGAGC
AGGTGGTAATTGGTGGTAATGAACGATACGTTTCTGTATGCCGTAAACACTATAAAG
AGGCGTTACAAGTCGACTCATTAACGGCTATTCAGGAAAGGCATCGCCACGATTAA

In another preferred embodiment, the protein encoded by the tdk gene has the following sequence (SEQ ID NO: 6; 205 amino acids, molecular weight 23456.2 Da):

MAQLYFYYSAMNAGKSTALLQSSYNYQERGMRTVVYTAEIDDRFGAGKVSS
RIGLSSPAKLFNQNSSLFDEIRAEHEQQAIHCVLVDECQFLTRQQVYELSEVVDQLDIP
VLCYGLRTDFRGELFIGSQYLLAWSDKLVELKTICFCGRKASMVLRLDQAGRPYNEG
EQVVIGGNERYVSVCRKHYKEALQVDSLTAIQERHRHD

In a preferred embodiment, said second regulatory sequence is inducible and capable of controlling expression of said second marker gene. In another preferred embodiment, said second regulatory sequence is a chemically or physically inducible promoter. In another preferred embodiment, said second regulatory sequence is inducible by the presence or absence of a compound selected from the group consisting of alcohols, antibiotics, steroids, metals, and saccharides. Said saccarides are preferably monosaccharides or disaccharides. In another preferred embodiment, said second regulatory sequence is inducible by lactose or isopropyl-β-D-1-thiogalactopyranoside (IPTG) or arabinose. In another preferred embodiment, said second regulatory sequence is inducible by tetracycline or a variant of tetracycline selected from the group consisting of anhydrotetracycline, minocycline, metacycline, sanocycline, demeclocycline, chloro-tetracycline, oxytetracycline, doxycycline, and tigecycline. In a certain preferred embodiment, said tdk gene is placed under the repression of lacI.

In a preferred embodiment, said tpm vector transferred in step (a) comprises said second marker gene, and (i) said first nucleotide sequence, and (ii) said second nucleotide sequence; and (iii) optionally said third nucleotide sequence;
wherein said method of the invention comprises the further steps of:
(d) culturing the selected bacterial host cells of step (c) that survived the tellurite concentration of step (b) and that have the vector integrated into their genome in medium, and
(e) counter-selecting from the cultured cells of step (d), bacterial host cells in which said flanked DNA sequence in the bacterial genome is eliminated from the genome, and/or bacterial host cells in which said optional third sequence is integrated in the genome of the counter-selected bacterial host cells.

In a preferred embodiment, said selected bacterial host cells are cultured in step (d) in a medium containing isopropyl-beta-D-1-thiogalactopyranoside (IPTG) and the second marker gene is placed under repression of lacI.Preferably said second marker gene is tdk placed under repression of lacI.

In a preferred embodiment, said counter-selecting of step (e) is preceded by culturing the selected cells in a medium containing azidothymidine (AZT). In a preferred embodiment, said selected bacterial host cells are cultured in step (d) in a medium containing AZT. In another preferred embodiment, said selected bacterial host cells are cultured in step (d) in a medium containing AZT and IPTG. In another preferred embodiment, said selected bacterial host cells are cultured in step (d) first in a medium containing AZT and then in a medium containing IPTG.

In a certain preferred embodiment, said tellurite containing medium of step (b) and/or said medium of step (d) contains a compound selected from the group consisting of a bicyclic compound substituted with at least one hydroxyl group or at least one amino group; a branched fatty acid having a chain length of more than C13; and an unsaturated fatty acid having a chain length of more than C14.

In another preferred embodiment, said medium of step (b) and/or said medium of step (d) contains a bicyclic compound substituted with at least one hydroxyl group or at least one amino group. In another preferred embodiment, said medium of step (b) and/or said medium of step (d) contains a branched fatty acid having a chain length of more than C13. In another preferred embodiment, said medium of step (b) and/or said medium of step (d) contains an unsaturated fatty acid having a chain length of more than C14.

In certain preferred embodiment, said bicyclic compound is selected from the group consisting of hydroxy-indole, hydroxy-naphthalene, and amino-naphthalene. In certain preferred embodiment, said bicyclic compound is selected from the group consisting of 4-hydroxyindol, 5-hydroxyindol, 6-hydroxyindol, 7-hydroxyindol, 1-naphtol, 2-naphtol, 1,2-dihydroxynaphtalene, 1,3-dihydroxynaphtalene 1,4-dihydroxynaphtalene, 2,3-dihydroxynaphtalene, 1-amino-naphthalene, and 2-amino-naphthalene.

In a further preferred embodiment, said branched fatty acid has a carbon chain length of C14 to C35, preferably C14 to C30, more preferably C14 to C25, again more preferably C14 to C20, and most preferably C15 to C17. In a further preferred embodiment, said branched fatty acid is selected from the group consisting of 12-methyltridecanoic acid (iso-C14:0), 12-methyltetradecanoic acid (anteiso-C15:0), 13-methyltetradecanoic acid (iso-C15:0), 14-methylpentadecanoic acid (iso-C16:0), 14-methylhexa-decanoic acid (anteiso-C17:0), and 15-methylhexadecanoic acid (iso-C17:0).

In a further preferred embodiment, said unsaturated fatty acid has a carbon chain length of C15 to C35, preferably C15 to C30, more preferably C15 to C25, again more preferably C15 to C20, and most preferably C16 to C18. In a further preferred embodiment, said unsaturated fatty acid is selected from the group consisting of 9-hexadecenoic acid (palmitoleic acid, C16:1), cis-9-octadecenoic acid (oleic acid, C18:1), cis-11-octadecenoic acid (vaccenic acid, C18:1), cis-9, and cis-12-octadecadienoic acid (linoleic acid, C18:2).

In a further aspect, the invention relates to a method for selecting bacterial host cells comprising the steps of:
(a) transferring a vector into bacterial host cells, wherein said vector comprises a marker gene,
(b) culturing the bacterial host cells in medium containing a compound selected from the group consisting of a bicyclic compound substituted with at least one hydroxyl group or at least one amino group; a branched fatty acid having a chain length of more than C13; and an unsaturated fatty acid having a chain length of more than C14, and
(c) isolating bacterial host cells that express said marker gene or that do not express said marker gene.

Clinical isolates of *Pseudomonas* and *Enterobacteriaceae* may be very motile due to the expression of different virulence factors, such as pili, or fimbriae. The motility of these clinical strains may be very problematic during the selection process of the genome editing procedure, because no clearly defined single colonies form and the colonies "swarm" into each other. Dependent on the motility of the strain, single clone formation can be promoted by inhibition of the cellular motility by addition of a compound into the medium, wherein said compound is selected from the group consisting of a bicyclic compound substituted with at least one hydroxyl group or at least one amino group; a branched fatty acid having a chain length of more than C13; and an unsaturated fatty acid having a chain length of more than C14.

In a certain preferred embodiment, said bacterial host cells are from of the genera *Escherichia* or *Pseudomonas.* In a further preferred embodiment, said bacterial host cells are from of the genera *Pseudomonas.* In an again more preferred embodiment, said bacterial host cells are *Pseudomonas aeruginosa.* Preferably, said bacterial host cells are from a strain that is resistant against antibiotics.

Again more preferably, said bacterial host cells are from a strain that is resistant against multiple antibiotics.

Preferably, said vector of the invention is transferred into the bacterial host cells via conjugation. Thus, in a preferred embodiment, said bacterial host cells are conjugative. In a preferred embodiment, said vector of the invention comprises an origin of transfer (oriT). The oriT is required for the plasmid transfer via conjugation.

In a preferred embodiment, said medium of step (b) contains a bicyclic compound substituted with at least one hydroxyl group or at least one amino group. In another preferred embodiment, said medium of step (b) contains a branched fatty acid having a chain length of more than C13. In another preferred embodiment, said medium of step (b) contains an unsaturated fatty acid having a chain length of more than C14.

In a further preferred embodiment, said bicyclic compound is selected from the group consisting of hydroxy-indole, hydroxy-naphthalene and amino-naphthalene. In another preferred embodiment, said bicyclic compound is selected from the group consisting of 4-hydroxyindol, 5-hydroxyindol, 6-hydroxyindol, 7-hydroxyindol, 1-naphtol, 2-naphtol, 1,2-dihydroxynaphtalene, 1,3-dihydroxynaphtalene 1,4-dihydroxynaphtalene, 2,3-dihydroxynaphtalene, 1-amino-naphthalene, and 2-amino-naphthalene.

In a further preferred embodiment, said branched fatty acid has a carbon chain length of C14 to C35, preferably C14 to C30, more preferably C14 to C25, again more preferably C14 to C20, and most preferably C15 to C17. In a further preferred embodiment, said branched fatty acid is selected from the group consisting of 12-methyltridecanoic acid (iso-C14:0), 12-methyltetradecanoic acid (anteiso-C15:0), 13-methyltetradecanoic acid (iso-C15:0), 14-methylpentadecanoic acid (iso-C16:0), 14-methylhexa-decanoic acid (anteiso-C17:0), and 15-methylhexadecanoic acid (iso-C17:0).

In a further preferred embodiment, said unsaturated fatty acid has a carbon chain length of C15 to C35, preferably C15 to C30, more preferably C15 to C25, again more preferably C15 to C20, and most preferably C16 to C18. In a further preferred embodiment, said unsaturated fatty acid is selected from the group consisting of 9-hexadecenoic acid (palmitoleic acid, C16:1), cis-9-octadecenoic acid (oleic acid, C18:1), cis-11-octadecenoic acid (vaccenic acid, C18:1), cis-9, and cis-12-octadecadienoic acid (linoleic acid, C18:2).

In a certain preferred embodiment, said bacterial host cells are *Pseudomonas aeruginosa* and said medium of step (b) contains 12-Methyl-tetradecanoic acid (C15).

In a further preferred embodiment, said medium contains said branched fatty acid or said unsaturated fatty acid, independently of each other, in a concentration of more than about 2 µg/ml, preferably in a concentration of about 5 µg/ml or more, more preferably in a concentration of about 10 µg/ml or more. In a further preferred embodiment, said medium contains said bicyclic compound in a concentration of more than about 5 µM, preferably in a concentration of about 50 µM or more, more preferably in a concentration of about 250 µM or more, and again more preferably in a concentration of about 500 µM or more.

In a certain preferred embodiment, said bacterial host cells are *Pseudomonas aeruginosa* and said medium of step (b) contains 10 µg/ml of 12-Methyl-tetradecanoic acid (C15).

The invention will now be illustrated by the following non-limiting examples.

### EXAMPLES

### EXAMPLE 1 - Constructions of gene deletions in multidrug-resistant Pseudomonas aeruginosa using two-step homologous recombination approach

*Pseudomonas aeruginosa* strains: Five different clinical isolates including multi-drug resistant (MDR) isolates as well as isolates of *Pseudomonas aeruginosa* (*P. aeruginosa*) PA-14 which is a widely used MDR P. aeruginosa model were used.

Vector design: The target DNA sequences (target genes) were lectin lecB, which was knocked out; mexR, which was mutated to T130P; and PA14_22730, which was mutated to T163P.

Cloning of target specific gene flanking sites to create a knock-out vector: The up- and downstream flanking sequences of these target genes or of the site to be modified were amplified by PCR (e.g., PCR-amplification of 700 bp, 500bp or 300 bp lecB upstream flanking region from PAO1 using primer pair oCK395+393, amplification of 700 bp, 500 bp or 300 bp lecB downstream flanking region from PAO1 using oCK384+385). Vector up- and downstream flanking sequences produced by this way have each a length of 700 bp, 500bp or 300 bp for knock-out. Site directed mutagenesis was performed by using 1x700 bp integration sequence including one of the mentioned mutations in the middle. The resulting PCR fragments were analyzed on agarose gels, connected (e.g., by size overlap extension PCR with both fragments as template and primer pair oCK395+385 to connect both lecB flanking sites). Knock-out cloning vector pCK348 and the PCR produced sequences were digested, e.g. by using EcoRI+XbaI, and ligated of the flanking sites into a plasmid producing a knock-out vector, e.g. pCK343 for lecB (by either standard restriction/digestion cloning or 1-step Gibson assembly) sites. The resulting vector was transformed into *E. coli* DH5alpha, *E. coli* S17 (or MFDpir) and selection on LB-kanamycin plates for plasmid propagation and analysis.

Conjugation: After confirmation of the correct plasmid, the plasmids were isolated and introduced into the conjugative *E. coli* strain MFDpir, which is auxotroph for diaminopimelic acid (DAP). The transfer of the plasmid in P. aeruginosa isolates was achieved by conjugation. 0.2-ml overnight cultures from the donor (MFDpir cells containing the knock-out plasmid) and receiver (P. aeruginosa isolate) strains were mixed. The cells were resuspended in 50µl of medium, transferred onto a 0.45µm-pore-size nitrocellulose filter placed on LB agar containing 300µM DAP and incubated 4h to overnight at 37°C for conjugative plasmid transfer.

Exemplary conjugation protocol: We grew *E. coli* S17 containing pCK343 (donor cells) and MDR-P. aeruginosa BV93 (receiver cells) in 2 ml LB-medium o/n @ 37°C, on the next day, we mixed 0.2 ml of donor and receiver cells in a 2 ml Eppendorf tube containing 1.2 ml LB-medium, collected cells by centrifugation at RT, 6000xg for 3min, resuspended the pellet with 50 µl LB-medium, placed a sterile 45um cellulose nitrate filter (Sartorius Stedim) on an LB-agar plate, applied the cell suspension containing donor and receiver cells on the surface of the filter, and incubated @ 37°C for 4h to overnight.

Non-antibiotic selection: After overnight incubation, the cells were scraped off the filter and resuspended in 0.4 ml of 0.85% (wt/vol) NaCl. Aliquots were plated on LB agar plates containing 100-400 µg/ml sodium tellurite. The concentration of tellurite was dependent on the clinical isolate. Optionally chloramphenicol is added. Tellurite selects for transgenic P. aeruginosa, whereas chloramphenicol kills the *E. coli* cells. Alternatively, *E. coli* MFDpir can be used in the conjugation as donor cells (auxotroph for diaminopimelic acid (DAP), which can be killed by depletion of DAP from the selection medium). Plates were incubated at 37°C for 24-48h to select for plasmid integration. Clones were screened for genomic plasmid integration by PCR.

Colony PCR screen to test site directed plasmid integration into genome: Single clones were picked from the Tellurite plate, resuspended in 10 µL MilliQ water, 1µL of cell suspension were spotted on Chromagar orientation (quality control, cells should be creamy translucent for P. aeruginosa) and LB plate containing 100-400µg/mL Tellurite (Masterplate). Plates were incubated at 37°C, and 1µL of cell suspension as DNA-template was used to perform a colony PCR clone screen. The resulting PCR fragments are analyzed on agarose gels (Fig. 8A).

The first PCR primer used for screening for genomic plasmid integration binds directly before the upstream regulatory sequence used. The 2nd PCR primer binds inside the integrated vector. PCR products were only obtained when the vector is integrated into the genome, and it can be distinguished if the vector is integrated up- or downstream of the target gene.

| **PCR reaction** | **PCR parameter** |
|---|---|
| 10µL OneTaq 2xMM (New England Biolabs, NEB) | 95°C 2:00min |
| | 95°C 0:30min |
| 1µL Primer 1 (oCK354, 10uM) | 52°C 0:30min |
| 1µL Primer 2 (oCK391, 10uM) | 68°C 1:40min Repeat 2-4 30x |
| 7µL MilliQ water | 68°C 5:00min |
| 1µL cell suspension (Template) | 4°C hold |

Counter-selection with inducible thymidine kinase to remove plasmid from genome of P. aeruginosa: Clones containing up- and/or downstream plasmid integrations were used to inoculate fresh LB broth containing 1 mM isopropyl-beta-D-1-thiogalactopyranoside (IPTG) and cultured for 2-3 h at 37°C to express the heterologous thymidine kinase tdk. Culture aliquots of a 1:10 dilution series were plated on LB agar plates containing 1mM IPTG, 200µg/ml azidothymidine (AzT) and 10µg/mL 2-methyltetradecanoic acid (C15) and incubated overnight at 37°C for plasmid removal from the genome. Clones were screened for gene deletion and plasmid removal by PCR. The genomic gene deletions were finally confirmed by DNA sequencing (Microsynth AG, Balgach, Switzerland).

Colony PCR screen to test for genomic plasmid removal and confirm successful lecB gene knock-out: We picked single clones from LB-AzT plate, resuspended it in 10 µL MilliQ water, spotted 1µL of cell suspension on CHROMagar Orientation (QC, PAO should be creamy translucent) and LB-Tellurite (here cells with removed plasmid will not grow (positive control), incubated the plate at 37 °C for 16-24h, used 1µL of cell suspension as DNA-template to perform a colony PCR clone screen, afterwards, separated DNA fragments on an agarose gel (Fig. 8B, C).

| **PCR reaction:** | **PCR parameter:** |
|---|---|
| 10µL OneTaq 2xMM (New England Biolabs, NEB) | 95°C 2:00min |
| | 95°C 0:30min |
| 1µL Primer 1 (oCK389 or oCK395 10uM) | 52°C 0:30min |
| 1µL Primer 2 (oCK390 or oCK385, 10uM) | 68°C 2:00min Repeat 2-4 30x |
| 7µL MilliQ water | 68°C 5:00min |
| 1µL cell suspension (Template) | 4°C hold |

Each of clones 2, 5, 6, 7, and 8 showed successful deletion of lecB and genomic plasmid removal (1.5 kb). In contrast, clones 1, 3, and 4 were wildtype revertants and kept the lecB gene, similar than the parental BV93 control strain.

PCR fragments (PCR oCK395+385) of positive clones were excised from the agarose gel, gel purified and analyzed by Sanger DNA-sequencing using oCK395 and oCK385 to confirm the successful deletion of the target gene lecB from the bacterial genome.

Gene knock-ins were produced by the same method with a vector that carries the DNA fragment to be inserted in-between the vector flanking sequences.

### Primer sequences used for PCRs (Example 1)

| **Primer** | **Comment** | **Sequence (5' - 3')** |
|---|---|---|
| **oCK354 SEQ ID NO: 7** | 3' primer, anneals in T0 of the knock-out plasmid | CCGAGCGTTCTGAACAAATC |
| **oCK384 SEQ ID NO: 8** | 5' primer, amplification of lecB downstream region from PAO1, **adds overlapping nucleotides from upstream region and SmaI** | GGTATTCAGTGGAGATACACCC GGGAGTTCGGAAGGACGGGATG |
| **oCK385 SEQ ID NO: 9** | 3' primer, amplification of lecB downstream region from PAO1 for clean KO, adds Xbal | gctctagaTGTCGCGGAAGCGATGA AG |
| **oCK389 SEQ ID NO: 10** | 5' primer, amplification of lecB for genotyping | ATGGCAACACAAGGAGTGTTC |
| **oCK390 SEQ ID NO: 11** | 3' primer, amplification of lecB for genotyping | AGCGGCCAGTTGATCACCAC |
| **oCK391 SEQ ID NO: 12** | 5' primer, for screening of lecB KO / plasmid integration, anneals upstream of 700 bp lecB flanking region | GCTTCCTCCAGTGCCAGTTC |
| **oCK393 SEQ ID NO: 13** | 3' primer, amplification of lecB upstream region from PAO1 (BV34) and PA14 (BV236), adds SmaI | tcccccgGGTGTATCTCCACTGAATA CC |
| **oCK395 SEQ ID NO: 14** | 5' primer, amplification of lecB upstream region from PAO1 (BV34), adds StuI-EcoRI | aaggcctggaattcGGATCGAGGCGGAA CAATAC |

### EXAMPLE 2: Enterobacteriaceae knock-outs, knock-ins and site directed mutagenesis using two-step homologous recombination approach

Enterobacteriaceae strains: Experiments were carried out with *Escherichia coli (E. coli), Klebsiella pneumoniae (K. pneumoniae)* or *Shigella flexneri (S. flexneri).* The modified strains include uropathogenic *E. coli* (UPEC), adherent invasive *E. coli* (AIEC) and laboratory *E. coli* strains and different drug resistant clinical isolates of *K. pneumoniae* and S. flexneri.

Vector design: The target DNA sequences (target genes) were envZ gene or ompR gene or both. These genes were knocked out or double knocked out. Vector up- and downstream flanking sequences produced by this way have each a length of 700 bp or less (700 bp, 500bp or 300 bp).

Cloning of target specific gene flanking sites into cloning vector pCK452, creating knock-out vector pCK476: The up- and downstream flanking sequences of these target genes were amplified by PCR. In detail, 700 bp, 500bp or 300 bp OmpR_KP upstream flanking region from BV318 were amplified using primer pair oCK612+613, and 700 bp, 500bp or 300 bp OmpR_KP downstream flanking region from BV318 were amplified using oCK614+615.

| **PCR reaction** | **PCR parameter** |
|---|---|
| 12.5 µL OneTaq 2xMM (New England Biolabs, NEB) | 95°C 2:00 min |
| | 95°C 0:30 min |
| 1 µL Primer 1 (10µM) | 54°C 0:30 min |
| 1 µL Primer 2 (10µM) | 68°C 0:40 min Repeat 2-4 30x |
| 0.5 µL genomic DNA (Template) | 68°C 5:00 min |
| 10.5 µL MilliQ water | 4°C hold |

The resulting PCR fragments are analyzed on agarose gels (1% (w/v) Tris acetate EDTA (TAE) buffered agarose gel for 30min at 130V) (Fig. 6A). The obtained PCR products were excised from the gel and gel purified using Qiaquick gel extraction kit (Qiagen) according to the manufacturer's protocol.

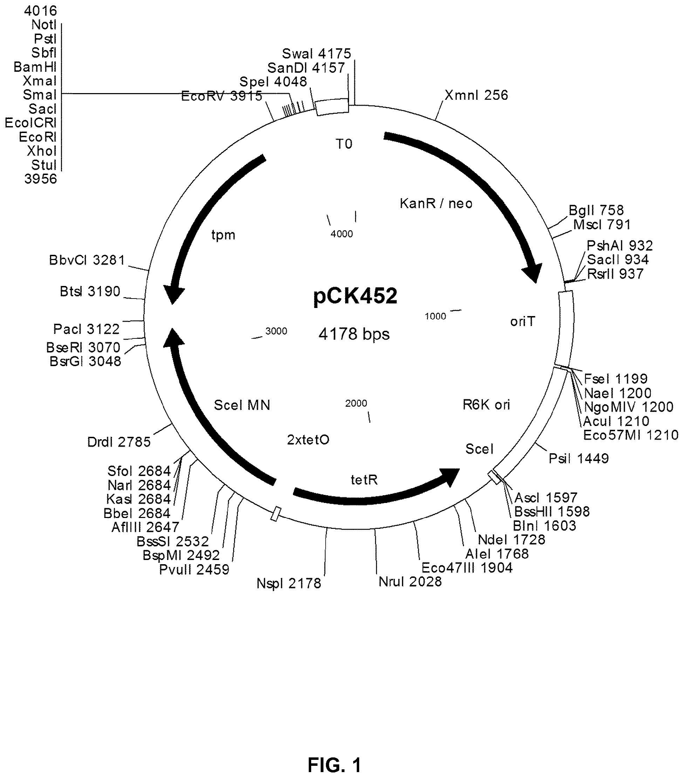

Vector pCK452 (see Fig. 1) was digested overnight at 37°C with KpnI+SpeI. The opened vector was separated on a 1% (w/v) TAE-agarose gel for 30min at 130V, the DNA was excised from the gel and gel purified using Qiaquick gel extraction kit (Qiagen) according to the manufacturer's protocol.

Restriction digestion of pCK452: 10µL pCK452 (1µg), 2.5µL 10x Cutsmart buffer (NEB), 1µL KpnI-HF (NEB), 1µL SpeI-HF (NEB), 10.5µL water.

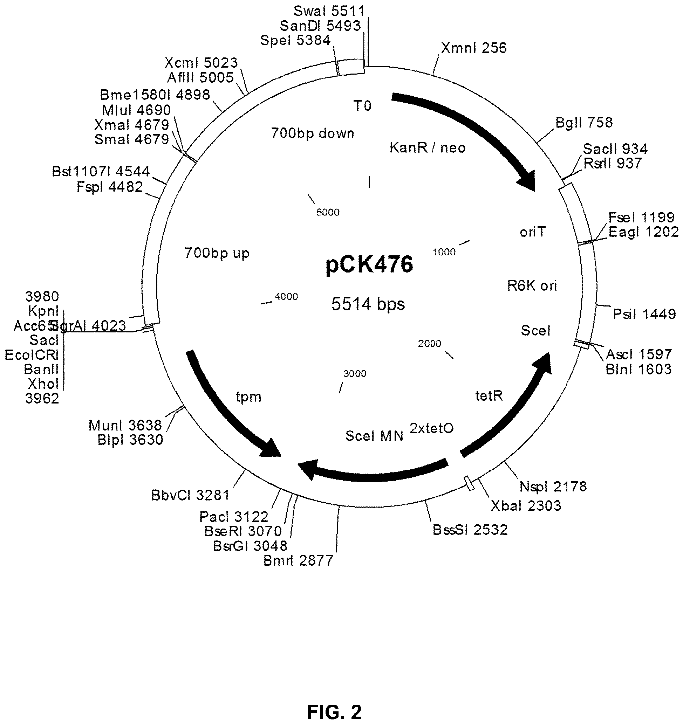

Afterwards the DNA fragments (up-, downstream flanking region and digested pCK452) were assembled either by standard restriction/digestion cloning or by 1-step Gibson assembly, e.g. into the EcoRI/XbaI sites, obtain the ompR-knock-out vector pCK476 (Fig. 2).

Gibson reaction: 0.5 µL pCK452 (KpnI/SpeI-digested), 1.0 µL PCR product oCK612+613 (upstream flank), 1.0 µL PCR product oCK614+615 (downstream flank), 2.5 µL 2x NEBuilder® HiFi DNA Assembly (incubation for 30 minutes at 50 °C, transformation of 2µL of the Gibson assembly into *E. coli* MFDpir; auxotroph for diaminopimelic acid (DAP), able to replicate R6K-ori containing plasmids)

The vector pCK452 is based on pVT77, but carries the different origin of replication R6K, and the anhydrotetracycline-inducible I-SceI-meganuclease counter selection cassette. The resulting vector was transformed into *E. coli* PIR2 which carry the Pir-gene and allow plasmid propagation of vectors with R6K-oris for plasmid propagation and analysis.

Conjugation: After confirmation of the correct plasmid, the plasmids were isolated and introduced into the conjugative *E. coli* strain MFDpir, which is auxotroph for diaminopimelic acid (DAP). The transfer of the plasmid in *E. coli, K. pneumoniae,* or S. flexneri isolates was achieved by conjugation. 0.2-ml overnight cultures from the donor (MFDpir cells containing the knock-out plasmid) and receiver (*E. coli, K. pneumoniae,* or S. flexneri isolates) strains were mixed. The cells were resuspended in 50 µl of medium, transferred onto a 0.45 µm-pore-size nitrocellulose filter placed on LB agar containing 300 µM DAP and incubated overnight at 30°C for conjugative plasmid transfer.

Exemplary protocol for conjugation: We grew *E. coli* MFDpir containing pCK476 (donor cells) and *K. pneumoniae* ATCC-43816 (receiver cells) in 2 ml LB-medium (supplemented with 300uM DAP for donor cells) o/n @ 30 °C; next day mixed 0.2 ml of donor and receiver cells in a 2 ml Eppendorf tube containing 1.2 ml LB-medium, collected cells by centrifugation at RT, 6000xg for 3min, resuspended the pellet with 50 µl LB-medium, placed a sterile 0.45µm cellulose nitrate filter (Sartorius Stedim) on an LB-agar plate supplemented with 30µM DAP, applied the cell suspension containing donor and receiver cells on the surface of the filter, incubated @ 30°C for 4h to overnight.

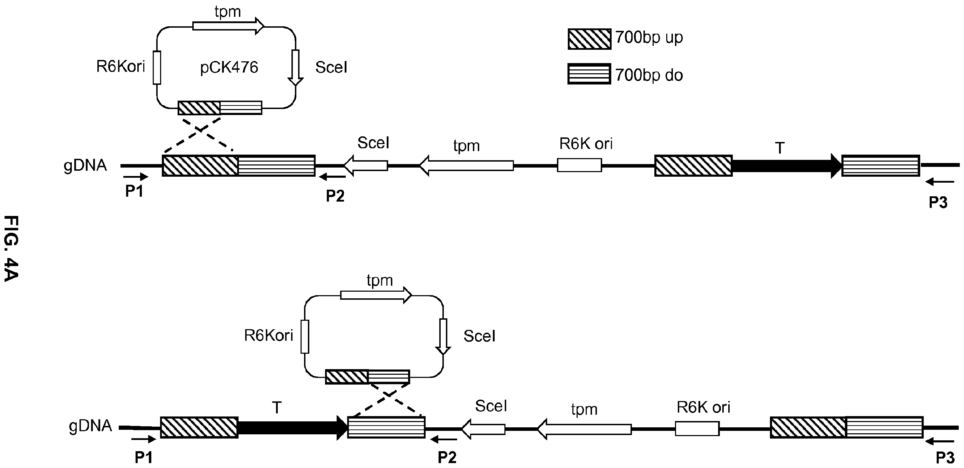

Non-antibiotic selection: After incubation, the cells were scraped off the filter and resuspended in 0.4 ml of 0.85% (wt/vol) NaCl, and 0.1-ml aliquots were plated on LB agar plates containing 100-400 µg/ml sodium tellurite or more (no DAP was used to kill auxotroph donor strains *E. coli* MFDpir). The concentration of tellurite depended on the clinical isolate. After overnight selection at 30°C, clones were screened for genomic plasmid integration by PCR (Fig. 4A left and right).

### Colony PCR screen to test site directed plasmid integration into genome

We picked single clones from Tellurite plate, suspended them in each 10 µL MilliQ water, spotted 1µL of cell suspension on Chromagar orientation (quality control, cells should be dark blue for *K. pneumoniae*) and LB plate containing 100ug/mL Tellurite (Masterplate). Plates were incubated plates at 30°C. 1µL of cell suspension was used as DNA-template to perform a colony PCR clone screen. After performing the PCR, the DNA fragments were separated on an agarose gel (Fig. 6B).

| **PCR reaction** | **PCR parameter** |
|---|---|
| 10µL OneTaq 2xMM (New England Biolabs, NEB) | 95°C 2:00min |
| | 95°C 0:30min |
| 1µL Primer 1 (oCK354, 10uM) | 55°C 0:30min |
| 1µL Primer 2 (oCK454, 10uM) | 68°C 2:30min Repeat 2-4, 30x |
| 7µL MilliQ water | 68°C 5:00min |
| 1µL cell suspension (Template) | 4°C hold |

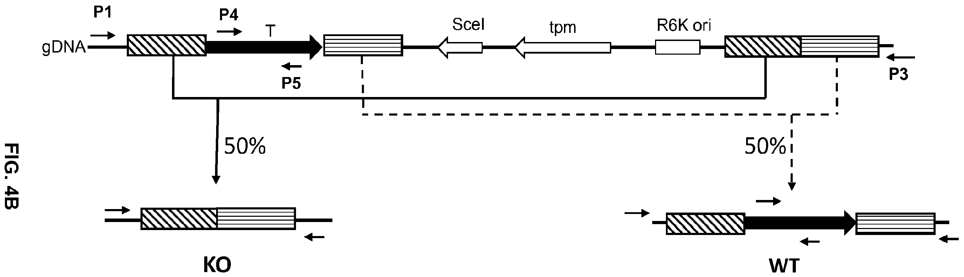

Counter-Selection with SceI to remove plasmid from genome (Fig. 4B, right): Clones containing up- and/or downstream plasmid integrations were dissolved in fresh LB broth and culture aliquots (0.1 ml) of a 1:10 dilution series were plated directly on LB agar plates containing 0.2, 2 and 20 µg/ml anhydrotetracycline (aTc; to induce the SceI-mediated counter-selection, inducer concentration can be adjusted for different strains) and incubated overnight at 30°C for plasmid removal from the genome.

Colony PCR screen to confirm successful plasmid removal: Clones were screened for gene deletion and plasmid removal by PCR. After performing the PCR, the DNA fragments were separated on an agarose gel (Fig. 6C). Clones 1, 3-6 show successful plasmid removal (1.5kb) and absence of ompR gene. In contrast, clone 2 is a wildtype revertant and still carries the ompR gene. The genomic gene deletions of the target gene ompR were finally confirmed by Sanger DNA-sequencing using oCK454 and oCK455 (Microsynth AG, Balgach, Switzerland). Stable knock-outs were produced by this method.

| **PCR reaction** | **PCR parameter** |
|---|---|
| 10µL OneTaq 2xMM (New England Biolabs, NEB) | 95°C 2:00min |
| | 95°C 0:30min |
| 1µL Primer 1 (oCK454 or oCK456, 10uM) | 52°C 0:30min |
| 1µL Primer 2 (oCK455 or oCK457, 10uM) | 68°C 2:00min Repeat 2-4, 30x |
| 7µL MilliQ water | 68°C 5:00min |
| 1µL cell suspension (Template) | 4°C hold |

### Primer sequences used for PCR (Example 2):

| **Primer** | **Comment** | **Sequence (5' - 3')** |
|---|---|---|
| **oCK354 SEQ ID NO: 7** | 3' primer, anneals in T0 of pSEVA vectors / pCK314 / pCK315, for screen of VanRa gene disruption | CCGAGCGTTCTGAACA AATC |
| **oCK454 SEQ ID NO: 15** | 5' primer, for screening of OmpR KO / plasmid integration, anneals upstream of 700 bp OmpR flanking region in *K. pneumoniae* | CGTCTCTCGGAAAGTTC TTG |
| **oCK455 SEQ ID NO: 16** | 3' primer, for screening of OmpR KO / plasmid integration, anneals downstream of 700 bp OmpR downstream flanking region in *K. pneumoniae* | TCGTAAATCATGACTG ACCC |
| **oCK456 SEQ ID NO: 17** | 5' primer, for OmpR KP genotyping, anneals in OmpR of *K. pneumoniae* | CGTCTGTTAACCCGTGA ATC |
| **oCK457 SEQ ID NO: 18** | 3' primer, for OmpR_KP genotyping, anneals in OmpR of *K. pneumoniae* | CGCTCCATCGCAGAGT ATTC |
| **oCK612 SEQ ID NO: 19** | 5' primer, amplification of OmpR upstream region from *Klebsiella pneumoniae* ATCC for clean KO, **adds KpnI and overhang with pCK452** | gaggaattcgagctcggtaccCG TATTCGATCTCGTTGAC ATAC |
| **oCK613 SEQ ID NO: 20** | 3' primer, amplification of OmpR upstream region from *Klebsiella pneumoniae* ATCC for clean KO, **adds** SmaI **and overhang with downstream flanking sequence** | cgcgtttcatcccgggTGTTTGT ACTCCCAAAGGTTCAC |
| **oCK614 SEQ ID NO: 21** | 5' primer, amplification of OmpR downstream region from *Klebsiella pneumoniae* ATCC for clean KO, **adds** SmaI **and overhang with upstream flanking sequence** | aacacccgggATGAAACGC GTGCGCTTTTCG |
| **oCK615 SEQ ID NO: 22** | 3' primer, amplification of OmpR downstream region from *Klebsiella pneumoniae* ATCC for clean KO, **adds SpeI and overhang with pCK452** | ctatcaacaggagtccaagactagt GTCATCAGCCAGCTGC TTCAC |

Gene knock-ins and site directed mutagenesis are also produced by this method using differently constructed vectors. For gene knock-ins, the DNA fragment is inserted into the vector between the vector target upstream and downstream flanking sites. For site directed mutagenesis only one integration site (1x700 bp or less) is included in the vector, which carries the nucleotides to be modified in the middle (see Fig. 3).

### EXAMPLE 3: Promotion of single clone screen via inhibition of the motility of P. aeruginosa clinical strains by 12-Methyl-tetradecanoic acid (C15)

Motility of clinical *P. aeruginosa* strains was inhibited by 12-Methyl-tetradecanoic acid (C15). Three different clinical isolates of *P. aeruginosa* were plated on LB-agar plates in the absence (Fig. 9, left) and presence (Fig. 9, right) of 10 µg/ml C15 to inhibit cellular motility. The plates were incubated for 16h at 37°C. The presence of C15 inhibited the cellular motility, leading to small well-defined colonies. In contrast, in the absence of C15, the colonies are diffuse and swarm into each other, making the selection of distinct single colonies in the knock-out screening process very difficult.

## Claims

1. A vector for manipulation of a genome of a bacterium, wherein said vector comprises
(a) at least one non-antibiotic selection marker gene cassette comprising at least one non-antibiotic selection marker gene and a first regulatory sequence, wherein said first regulatory sequence is operatively linked to said at least one non-antibiotic selection marker gene,
(b) an origin of replication, wherein said origin of replication is not capable of inducing replication of said vector in said bacterium, and
(c) a restriction endonuclease gene, a recognition site of a restriction endonuclease encoded by said restriction endonuclease gene, and a second regulatory sequence, wherein said second regulatory sequence is operatively linked to said restriction endonuclease gene.

2. The vector according to claim 1 further comprising a nucleotide sequence consisting of
(i) a first nucleotide sequence, and
(ii) a second nucleotide sequence,
wherein the first nucleotide sequence is at least 80% identical to an up-stream flanking DNA sequence which flanks upstream a target DNA sequence in the bacterial genome, and the second nucleotide sequence is at least 80% identical to a down-stream flanking DNA sequence which flanks downstream said target DNA sequence in the bacterial genome, and wherein the first nucleotide sequence adjoins the second nucleotide sequence in the vector.

3. The vector according to claim 1 further comprising a nucleotide sequence consisting of
(i) a first nucleotide sequence,
(ii) a second nucleotide sequence, and
(iii) a third nucleotide sequence,
wherein in the vector said third nucleotide sequence is at one end flanked by said first nucleotide sequence and at the other end flanked by said second nucleotide sequence, and wherein the first nucleotide sequence is at least 80% identical to an up-stream flanking DNA sequence in the bacterial genome, and the second nucleotide sequence is at least 80% identical to a down-stream flanking DNA sequence in the bacterial genome, and
said upstream and said downstream flanking DNA sequences either directly flank each other in the bacterial genome or flank a DNA sequence located between the upstream and downstream flanking DNA sequence in the bacterial genome.

4. The vector of any one of the preceding claims, wherein said non-antibiotic selection marker gene is selected from the group consisting of a heavy metal resistance gene, triclosan resistance gene, glyphosate resistance gene, bialaphos resistance gene, and phosphinothricin resistance gene.

5. The vector of any one of the preceding claims, wherein said non-antibiotic selection marker gene is a heavy metal resistance gene selected from the group consisting of an arsenic resistance gene, tellurite resistance gene, and mercury resistance gene.

6. The vector of any one of the preceding claims, wherein said non-antibiotic selection marker gene is a tellurite resistance gene, wherein preferably said tellurite resistance gene is thiopurine S-methyltransferase (tpm).

7. The vector of any one of the preceding claims, wherein said non-antibiotic selection marker gene cassette has a length of less than 2000 bp, preferably less than 1000 bp, more preferably less than 700 bp, again more preferably about 650 bp.

8. The vector of any one of the preceding claims, wherein said restriction endonuclease is a member of the family of LAGLIDADG homing endonucleases.

9. The vector of claim 8, wherein said member of the family of LAGLIDADG homing endonucleases is I-SceI.

10. The vector of any one of the preceding claims, wherein said bacterium is a member of the *Enterobacteriaceae.*

11. The vector of any one of the preceding claims, wherein said bacterium is selected from the group consisting of the genera *Escherichia, Klebsiella, Shigella, Enterobacter, Yersinia, Salmonella, Serratia, Citrobacter,* and *Proteus.*

12. The vector of any one of the preceding claims, wherein said second regulatory sequence is a promoter inducible by tetracycline or by a variant of tetracycline selected from the group consisting of anhydrotetracycline, minocycline, metacycline, sanocycline, demeclocycline, chloro-tetracycline, oxytetracycline, doxycycline, and tigecycline.

13. A method for genetic manipulation of bacteria comprising the steps of:
(a) transferring the vector of claims 2 to 12 into bacterial host cells,
(b) culturing said bacterial host cells in a first medium,
(c) selecting from the cultured cells of step (b), bacterial host cells having the vector integrated into their genome,
(d) culturing the selected bacterial host cells of step (c) in a second medium, and
(e) counter-selecting from the cultured host cells of step (d), bacterial host cells in which said target DNA sequence is eliminated from the bacterial genome or in which said third nucleotide sequence is integrated into the bacterial genome.

14. The method of claim 13, wherein the vector is of claim 6, and said first medium of step (b) contains more than 100 µg/ml tellurite, preferably 200 µg/ml tellurite or more, more preferably 300 µg/ml tellurite or more, again more preferably 400 µg/ml tellurite or more.

15. The method of claim 13 or 14, wherein said first medium of step (b) and/or said second medium of step (d) contains a compound selected from the group consisting of a bicyclic compound substituted with at least one hydroxyl group or at least one amino group; a branched fatty acid having a chain length of more than C13; and an unsaturated fatty acid having a chain length of more than C14.


SEQUENCE LISTING

<110>  BioVersys AG

<120>  BACTERIAL VECTORS FOR GENETIC MANIPULATION OF BACTERIA

<130>  P5410EP00

<160>  22

<170>  PatentIn version 3.5

<210>  1
<211>  654
<212>  DNA
<213>  Acinetobacter baylyi

<400>  1
atgcaacatg agttctggca tcaacgctgg caggaaaaca gaatcggttt tcatcaattt      60

acaccgagtc cactgttagt cgattatttt aacgaacttg gcttaaaaac ctctgcccgt     120

atctttgttc cattgagtgg taaaacactg gatattagtt ggttattaca acaaggctac     180

catgttgtgg caattgagct tagccaaatc gctgtaacat cattgattga gcaattagtt     240

gaagacttcg atatacaatt tgagtcttcc gaaaagaata atctgatcca ttaccatcac     300

ccacaaattg atatttttgt cggtgatttt tttgatctaa gcaaagaaca attaggacag     360

gtcgatgcaa tttttgatcg tgcagcattg attgctttac cagacgacat acgccaagac     420

tatgttcaac acttgatcga gatcagtggt gctgcatctc agtttttaat tagctaccaa     480

tacgatgcag gcagtcatga aggacctcct ttttcggtca atgcagaaga aattaaacaa     540

ctttatgctg aggcttatga catccgactt ttgaaggagc aacttgtcga tgcatctcaa     600

aacaaaggaa atcaccccaa gagcacacta tggatactca ctgccaagtt ctga           654

<210>  2
<211>  217
<212>  PRT
<213>  Acinetobacter baylyi

<400>  2

Met Gln His Glu Phe Trp His Gln Arg Trp Gln Glu Asn Arg Ile Gly
1               5                   10                  15

Phe His Gln Phe Thr Pro Ser Pro Leu Leu Val Asp Tyr Phe Asn Glu
            20                  25                  30

Leu Gly Leu Lys Thr Ser Ala Arg Ile Phe Val Pro Leu Ser Gly Lys
        35                  40                  45

Thr Leu Asp Ile Ser Trp Leu Leu Gln Gln Gly Tyr His Val Val Ala
    50                  55                  60

Ile Glu Leu Ser Gln Ile Ala Val Thr Ser Leu Ile Glu Gln Leu Val
65                  70                  75                  80

Glu Asp Phe Asp Ile Gln Phe Glu Ser Ser Glu Lys Asn Asn Leu Ile
                85                  90                  95

His Tyr His His Pro Gln Ile Asp Ile Phe Val Gly Asp Phe Phe Asp
            100                 105                 110

Leu Ser Lys Glu Gln Leu Gly Gln Val Asp Ala Ile Phe Asp Arg Ala
        115                 120                 125

Ala Leu Ile Ala Leu Pro Asp Asp Ile Arg Gln Asp Tyr Val Gln His
    130                 135                 140

Leu Ile Glu Ile Ser Gly Ala Ala Ser Gln Phe Leu Ile Ser Tyr Gln
145                 150                 155                 160

Tyr Asp Ala Gly Ser His Glu Gly Pro Pro Phe Ser Val Asn Ala Glu
                165                 170                 175

Glu Ile Lys Gln Leu Tyr Ala Glu Ala Tyr Asp Ile Arg Leu Leu Lys
            180                 185                 190

Glu Gln Leu Val Asp Ala Ser Gln Asn Lys Gly Asn His Pro Lys Ser
        195                 200                 205

Thr Leu Trp Ile Leu Thr Ala Lys Phe
    210                 215

<210>  3
<211>  702
<212>  DNA
<213>  Artificial Sequence

<220>
<223>  I-SceI vector

<400>  3
atgcatcaaa aaaaccaggt aatgaacctg ggtccgaact ctaaactgct gaaagaatac      60

aaatcccagc tgatcgaact gaacatcgaa cagttcgaag caggtatcgg tctgatcctg     120

ggtgatgctt acatccgttc tcgtgatgaa ggtaaaacct actgtatgca gttcgagtgg     180

aaaaacaaag catacatgga ccacgtatgt ctgctgtacg atcagtgggt actgtccccg     240

ccgcacaaaa aacaacgtgt taaccacctg ggtaacctgg taatcacctg gggcgcccag     300

actttcaaac accaagcttt caacaaactg gctaacctgt tcatcgttaa caacaaaaaa     360

accatcccga acaacctggt tgaaaactac ctgaccccga tgtctctggc atactggttc     420

atggatgatg gtggtaaatg ggattacaac aaaaactcta ccaacaaatc gatcgtactg 480

aacacccagt ctttcacttt cgaagaagta gaatacctgg ttaagggtct gcgtaacaaa 540

ttccaactga actgttacgt aaaaatcaac aaaaacaaac cgatcatcta catcgattct 600

atgtcttacc tgatcttcta caacctgatc aaaccgtacc tgatcccgca gatgatgtac 660

aaactgccga acactatctc ctccgaaact ttcctgaaat aa 702

<210> 4
<211> 233
<212> PRT
<213> Artificial Sequence

<220>
<223> I-SceI protein

<400> 4

Met His Gln Lys Asn Gln Val Met Asn Leu Gly Pro Asn Ser Lys Leu
1               5                   10                  15

Leu Lys Glu Tyr Lys Ser Gln Leu Ile Glu Leu Asn Ile Glu Gln Phe
            20                  25                  30

Glu Ala Gly Ile Gly Leu Ile Leu Gly Asp Ala Tyr Ile Arg Ser Arg
        35                  40                  45

Asp Glu Gly Lys Thr Tyr Cys Met Gln Phe Glu Trp Lys Asn Lys Ala
    50                  55                  60

Tyr Met Asp His Val Cys Leu Leu Tyr Asp Gln Trp Val Leu Ser Pro
65                  70                  75                  80

Pro His Lys Lys Gln Arg Val Asn His Leu Gly Asn Leu Val Ile Thr
                85                  90                  95

Trp Gly Ala Gln Thr Phe Lys His Gln Ala Phe Asn Lys Leu Ala Asn
            100                 105                 110

Leu Phe Ile Val Asn Asn Lys Lys Thr Ile Pro Asn Asn Leu Val Glu
        115                 120                 125

Asn Tyr Leu Thr Pro Met Ser Leu Ala Tyr Trp Phe Met Asp Asp Gly
    130                 135                 140

Gly Lys Trp Asp Tyr Asn Lys Asn Ser Thr Asn Lys Ser Ile Val Leu
145                 150                 155                 160

Asn Thr Gln Ser Phe Thr Phe Glu Glu Val Glu Tyr Leu Val Lys Gly

165                 170                 175

Leu Arg Asn Lys Phe Gln Leu Asn Cys Tyr Val Lys Ile Asn Lys Asn
            180                 185                 190

Lys Pro Ile Ile Tyr Ile Asp Ser Met Ser Tyr Leu Ile Phe Tyr Asn
        195                 200                 205

Leu Ile Lys Pro Tyr Leu Ile Pro Gln Met Met Tyr Lys Leu Pro Asn
    210                 215                 220

Thr Ile Ser Ser Glu Thr Phe Leu Lys
225                 230

<210>  5
<211>  618
<212>  DNA
<213>  Escherichia coli

<400>  5
atggcacagc tatatttcta ctattccgca atgaatgcgg gtaagtctac agcattgttg      60

caatcttcat acaattacca ggaacgcggc atgcgcactg tcgtatatac ggcagaaatt     120

gatgatcgct ttggtgccgg gaaagtcagt tcgcgtatag gtttgtcatc gcctgcaaaa     180

ttatttaacc aaaattcatc attatttgat gagattcgtg cggaacatga acagcaggca     240

attcattgcg tactggttga tgaatgccag tttttaacca gacaacaagt atatgaatta     300

tcggaggttg tcgatcaact cgatataccc gtactttgtt atggtttacg taccgatttt     360

cgaggtgaat tatttattgg cagccaatac ttactggcat ggtccgacaa actggttgaa     420

ttaaaaacca tctgtttttg tggccgtaaa gcaagcatgg tgctgcgtct tgatcaagca     480

ggcagacctt ataacgaagg tgagcaggtg gtaattggtg gtaatgaacg atacgtttct     540

gtatgccgta aacactataa agaggcgtta caagtcgact cattaacggc tattcaggaa     600

aggcatcgcc acgattaa                                                   618

<210>  6
<211>  205
<212>  PRT
<213>  Escherichia coli

<400>  6

Met Ala Gln Leu Tyr Phe Tyr Tyr Ser Ala Met Asn Ala Gly Lys Ser
1               5                   10                  15

Thr Ala Leu Leu Gln Ser Ser Tyr Asn Tyr Gln Glu Arg Gly Met Arg
            20                  25                  30

Thr Val Val Tyr Thr Ala Glu Ile Asp Asp Arg Phe Gly Ala Gly Lys
        35                  40                  45

Val Ser Ser Arg Ile Gly Leu Ser Ser Pro Ala Lys Leu Phe Asn Gln
    50                  55                  60

Asn Ser Ser Leu Phe Asp Glu Ile Arg Ala Glu His Glu Gln Gln Ala
65                  70                  75                  80

Ile His Cys Val Leu Val Asp Glu Cys Gln Phe Leu Thr Arg Gln Gln
                85                  90                  95

Val Tyr Glu Leu Ser Glu Val Val Asp Gln Leu Asp Ile Pro Val Leu
            100                 105                 110

Cys Tyr Gly Leu Arg Thr Asp Phe Arg Gly Glu Leu Phe Ile Gly Ser
        115                 120                 125

Gln Tyr Leu Leu Ala Trp Ser Asp Lys Leu Val Glu Leu Lys Thr Ile
    130                 135                 140

Cys Phe Cys Gly Arg Lys Ala Ser Met Val Leu Arg Leu Asp Gln Ala
145                 150                 155                 160

Gly Arg Pro Tyr Asn Glu Gly Glu Gln Val Val Ile Gly Gly Asn Glu
                165                 170                 175

Arg Tyr Val Ser Val Cys Arg Lys His Tyr Lys Glu Ala Leu Gln Val
            180                 185                 190

Asp Ser Leu Thr Ala Ile Gln Glu Arg His Arg His Asp
        195                 200                 205

<210>  7
<211>  20
<212>  DNA
<213>  Artificial Sequence

<220>
<223>  primer oCK354

<400>  7
ccgagcgttc tgaacaaatc                                                   20

<210>  8
<211>  45
<212>  DNA
<213>  Artificial Sequence

<220>
<223>  primer oCK384

<400>  8
ggtattcagt ggagatacac ccgggagttc ggaagggacg ggatg                    45


<210>  9
<211>  28
<212>  DNA
<213>  Artificial Sequence

<220>
<223>  primer oCK385

<400>  9
gctctagatg tcgcgggaag cgatgaag                                       28


<210>  10
<211>  21
<212>  DNA
<213>  Artificial Sequence

<220>
<223>  primer oCK389

<400>  10
atggcaacac aaggagtgtt c                                              21


<210>  11
<211>  20
<212>  DNA
<213>  Artificial Sequence

<220>
<223>  primer oCK390

<400>  11
agcggccagt tgatcaccac                                                20


<210>  12
<211>  20
<212>  DNA
<213>  Artificial Sequence

<220>
<223>  primer oCK391

<400>  12
gcttcctcca gtgccagttc                                                20


<210>  13
<211>  28
<212>  DNA
<213>  Artificial Sequence

<220>
<223>  primer oCK393

<400>  13
tcccccgggt gtatctccac tgaatacc                                       28

<210>  14
<211>  34
<212>  DNA
<213>  Artificial Sequence

<220>
<223>  primer oCK395

<400>  14
aaggcctgga attcggatcg aggcggaaca atac                                    34


<210>  15
<211>  20
<212>  DNA
<213>  Artificial Sequence

<220>
<223>  primer oCK454

<400>  15
cgtctctcgg aaagttcttg                                                    20


<210>  16
<211>  20
<212>  DNA
<213>  Artificial Sequence

<220>
<223>  primer oCK455

<400>  16
tcgtaaatca tgactgaccc                                                    20


<210>  17
<211>  20
<212>  DNA
<213>  Artificial Sequence

<220>
<223>  primer oCK456

<400>  17
cgtctgttaa cccgtgaatc                                                    20


<210>  18
<211>  20
<212>  DNA
<213>  Artificial Sequence

<220>
<223>  primer oCK457

<400>  18
cgctccatcg cagagtattc                                                    20


<210>  19

<211>  44
<212>  DNA
<213>  Artificial Sequence

<220>
<223>  primer oCK612

<400>  19
gaggaattcg agctcggtac ccgtattcga tctcgttgac atac                        44


<210>  20
<211>  39
<212>  DNA
<213>  Artificial Sequence

<220>
<223>  primer oCK613

<400>  20
cgcgtttcat cccgggtgtt tgtactccca aaggttcac                              39


<210>  21
<211>  31
<212>  DNA
<213>  Artificial Sequence

<220>
<223>  primer oCK614

<400>  21
aacacccggg atgaaacgcg tgcgcttttc g                                      31


<210>  22
<211>  46
<212>  DNA
<213>  Artificial Sequence

<220>
<223>  primer oCK615

<400>  22
ctatcaacag gagtccaaga ctagtgtcat cagccagctg cttcac                      46